# EUROPEAN PATENT SPECIFICATION

(11) **EP 2 323 702 B1**
(45) Date of publication and mention of the grant of the patent: **27.09.2017**
(21) Application number: 09776182.9
(22) Date of filing: 13.08.2009
(51) Int. Cl.: A61L 15/12

(54) **ORTHOPEDIC BANDAGE SET**
ORTHOPÄDISCHES VERBANDSSET
ENSEMBLE BANDAGE ORTHOPÉDIQUE

(30) Priority: 14.08.2008 DK 200801101
(43) Date of publication of application: 25.05.2011
(73) Proprietor: Straxfix. Technology IVS, 2900 Hellerup (DK)
(72) Inventor: JENSEN, Torben, Hove, DK-8270 Hojbjerg (DK)
(74) Representative: Patentgruppen A/S
(86) International application number: PCT/DK2009/000180
(87) International publication number: WO 2010/017817

(56) References cited:
- US-A- 3 421 501
- US-A- 3 881 473
- US-A1- 2005 233 149
- US-A1- 2006 221 617

## Description

### Scope of the invention

The invention relates in a 1st aspect to a set including a light hardenable (i.e. capable of hardening upon exposure to light) orthopedic bandage material and a lighting appliance which is suited for hardening such a material. The invention relates in a 2nd and 3rd aspect to a lighting appliance which is suited for hardening a light hardenable orthopedic bandage material, respectively a method for producing such a lighting appliance. Moreover, the invention relates in a 4th and 5th aspect to a light hardenable orthopedic bandage material, respectively a method for producing such a material. Furthermore, the invention relates in a 6th aspect to an arrangement including a lighting appliance according to the 1st, the 2nd or the 3rd aspect of the invention, and an electric power supply. Finally, the invention relates in a 7th aspect to applications of a lighting appliance according to the 1st, the 2nd or the 3rd aspect of the invention or an arrangement according to the 6th aspect of the invention for hardening a light hardenable orthopedic bandage material.

### Background of the invention

In what follows, 'orthopedic bandage' and 'bandage' will be used as general terms covering any device which is placed on or built up on the body or on a body member in human beings or animals in order to provide protection or physical support of the part of the body in question, or to limit its freedom of movement. Thus, 'orthopedic bandage' and 'bandage' will be understood to include such devices as orthopedic casts, splints and circular bandages as well as capsules, orthoses and spicae.

'Light' is to be understood in what follows as electromagnetic radiation in the ultraviolet, visible or infrared regions of the spectrum.

Orthopedic bandages used to be made of Plaster of Paris, which has a number of wellknown drawbacks such as a considerable demand for craftsmanship in the application of the bandage, a long setting time, messing of the working place, a high weight of the finished bandage, necessity of protecting the bandage against water, and poor X-ray penetrability. Plaster of Paris has therefore been extensively replaced by other bandage materials. Even bandage materials that can be hardened by exposure to light from special light sources have been proposed.

The use of sources of artificial light to evoke photochemical reactions for therapeutic, cosmetic, scientific or industrial purposes is wellknown, but in cases in which a body extending in three dimensions is to be exposed from several sides, use has hitherto been made of voluminous light cabinets constructed so as to surround the body wholly or partly, or of non-simultaneous exposure of the sides of the body, the body and the light source being turned or displaced stepwise or in a sliding manner in relation to each other.

Light hardening of workable substances is also commonly known. As an orthopedic bandage material, e.g. fabric impregnated with a light hardenable material has certain advantages over Plaster of Paris or fabric impregnated with Plaster of Paris such as cleanliness during application and subsequent removal of the bandagee, and low weight of the bandage in addition to good X-ray permeability; but in default of appropriate light sources and light hardenable bandage materials adapted to them, one of the most important advantages still remains to be obtained, namely the time saving by a short hardening process combined with handiness of the lighting equipment, for which reason light hardenable orthopedic bandage materials have reached no extension.

Among drawbacks of known light hardenable orthopedic bandage materials are the dependence on special chemical syntheses during their manufacture, content of volatile and/or harmful components such as styrene, requirement of use of protective gloves, and long hardening times.

Among drawbacks of known lighting appliances for the hardening of light hardenable orthopedic bandage materials are the emission of harmful ultraviolet radiation, big size and resulting unhandiness, e.g. when the lighting appliance has a number of tubular fluorescent lamps, and/or too strong development of heat, e.g. when the equipment has strong incandescent lamps. Since these drawbacks are unimportant in laboratory experiments, short hardening times may well be reached in such experiments, but these experiments do not realistically show what can be achieved using the same materials and lighting appliances in an operating room where the size and heat generation of the equipment are of great importance.

One particular circumstance may be problematic in some of the proposed methods for light hardening of bandages, namely the risk of zonal underhardening of the material by non-simultaneous exposure of different regions of the bandage. In photochemical hardening reactions, which pass through several steps with several possible reactions in which the intermediates have several possibilities of reaction of which not all lead to the desired crosslinks in the material, a better hardening is generally reached through a short and strong exposure than through a weak and long exposure. However, since an exposure which is too short or too weak to effect a satisfactory hardening implies a consumption of some of the reactive components, among which the photoinitiator necessary for the hardening reaction, a subsequent strong exposure will often not be capable of completing the hardening. Thus, e.g. a circular bandage which is exposed several times from one side at a time may become underhardened in those zones which during the first exposure receive the light at a low angle.

Summing up the drawbacks of the known combinations of light hardenable orthopedic bandage materials and lighting appliances, it can be said that they do not reach at the same time satisfactorily short hardening times and handiness of materials and equipment.

Almost universally used to replace Plaster of Paris are therefore, though less advantageous than the light hardenable bandage materials, chemically hardening materials containing organic isocyanates and thermoplastic materials.

The isocyanate containing materials harden by reaction with water or moisture from the air. The hardening starts as soon as the material is removed from the protective package, and the user only has a very limited influence on the hardening time. The latter is adjusted by the manufacturer, but cannot be made arbitrarily short since time is required to apply the bandage. Times of from 15 to 30 minutes from the opening of the package till complete hardening has taken place are common with these materials. In addition to this, the isocyanate content is undesirable for occupational health reasons, and the use of gloves is necessary in handling the unhardened bandage material as residues of the binder are left on the hands.

The thermoplastic bandage materials must be softened in a water bath at 60 - 70 degrees C, which is an unpleasantly high temperature for the person who is applying the bandage. The material hardens on cooling, and the cooling starts when the material is removed from the water bath. The application of the bandage therefore has to be completed quickly, typically within 3 minutes, but the bandage only reaches its full strength after almost complete cooling which typically takes 15 minutes.

Thus, on balance, a satisfactory successor for Plaster of Paris as an orthopedic bandage material has not been found prior to the present invention.

A selection of publications which mention light hardenable orthopedic bandage materials may include the following.

In US 3 985 128 (Garwood & Taw, 1975), unsaturated polyester resin with a content of styrene, which for health reasons is undesirable, is used as a light hardenable material. For the hardening use is made of ultraviolet light with a peak wavelength of 367 nm from 16 fluorescent tubes mounted in a cylindrical reflector having a diameter of 45 cm, i.e. both an undesirable type of light and a bulky equipment which is unrealistic to use in an operating room.

US 4 052 282 (Kubushiro, 1975) relates to a bandage material in which the light hardenable resin has to be synthesized as a part of the manufacture of the bandage material, which is quite laborious. The lighting equipment is not described well in all examples, but in one example use is made of a cylindrical lighting appliance having an internal diameter of 40 cm equipped with 20 ultraviolet fluorescent tubes, i.e. again an inconveniently big lighting appliance, to which must be added the fact that the exposure time necessary is 3 - 4 minutes.

In NL 1 001 552 (Glastra, 1995), the chemical system is not discussed in detail, but as possible lighting appliances are mentioned light cabinets with ultraviolet lamps, and detached ultraviolet lamps which are obviously unthinkable to use since staff and patients cannot be exposed to the radiation from such lamps. It is furthermore suggested to move an ultraviolet lamp around the bandage, which, however, is unlikely to give a proper hardening, cf. the above remarks on underhardening.

In WO 00/61692 (Ansell, 1999), the chemical system is described whereas the lighting equipment is hardly mentioned. For the production of the light hardenable system, the synthesis of a reactive polymer is required which is comparatively laborious. Exposure times of 1 - 15 minutes are mentioned almost as a suggestion, but are not claimed to have been attained.

In US 2008/0 045 622 A1 (Nakasugi & Matsumoto, 2005), use is made of a urethane (meth)acrylate oligomer as light hardenable constituent of the bandage material. In the publication, it is explicitly mentioned that protective gloves must be used when handling the bandage material which is a drawback. The exposure examples include use of fluorescent tubes or a strong halogen lamp, and exposure times of 40 seconds to 2 minutes are reached in hardening of laboratory specimens of the bandage material. An exposure time of 40 seconds has been reached using a 500 W halogen lamp at a distance of 20 cm. For the hardening of a whole bandage, one would have to imagine an arrangement including several built-in 500 W lamps which would again be unhandy and develop too strong heat.

US3881473 (Corvi et al) discloses a UV hardenable orthopedic bandage material for a limb which can be hardened by UV light.

### Description of the invention

As will have been understood from what has been said above, achieving the advantages of the light hardenable orthopedic bandage materials in practice requires the availability of a suitable bandage material which is non-harmful and convenient to handle, and the availability of a lighting appliance which is not unhandy as a result of its size, which is capable of lighting the whole surface of the bandage simultaneously, and which is capable of effecting within a short exposure time a complete hardening without excessive heat development and without giving off harmful radiation to staff or patient.

As will be seen from the description below, these requirements are met by the present invention, which in a 1 st aspect according to claim 1 relates to a lighting appliance which is suited for hardening a light hardenable orthopedic bandage material and which invention in a 2nd aspect according to claim 9 relates to an orthopedic bandage set including a light hardenable orthopedic bandage material and a lighting appliance which is suited for hardening such a material.

Thus, a preferred embodiment of the invention includes a light hardenable bandage material in the form of net impregnated with an adhesive but not residue-leaving substance, which can be used for building up bandages, e.g. on broken arms and legs, in a similar way that known replacement materials for Plaster of Paris are used, but which - as distinct from known materials - can be hardened in a very short time, e.g. 45 seconds, exactly when this is desired, just by exposing it to the light from the lighting appliance also included in the present invention. In a preferred embodiment the lighting appliance appears as a flexible, relatively thin shell or jacket with built-in light-emitting diodes, which by means of wires are connected to an electric power supply. When the relatively pliable bandage material has been placed on the part of the body in question and pressed gently down to follow the shape of the part of the body, the lighting appliance is placed against the bandage material and then switched on and kept switched on for a short time. After the exposure, the bandage material will appear hardened, and the bandage can be finished, cf. example 4.

Here follows a survey of the advantages of the light hardenable orthopedic bandage material and the lighting appliance according to the invention.

Over Plaster of Paris, the bandage material according to the invention has the following advantages:
- cleanly work during application of the bandage
- X-raying through the bandage possible with good results
- low weight of the finished bandage
- openness of the bandage (due to net structure or the like as distinct from solid Plaster of Paris, which is less pleasant to the person wearing the bandage)
- water-resistance of the bandage (so that the person wearing the bandage may take a shower)
- cleanly removal of the used bandage (no dust as with Plaster of Paris)

As opposed to the known systems offering replacement materials for Plaster of Paris, the bandage set according to the invention provides the following advantages:
- absence of harmful organic isocyanates (one of the drawbacks of moisture hardenable bandage materials)
- freedom from use of hot water bath (one of the drawbacks of thermoplastic bandage materials)
- possibility of handling the material without protective gloves
- unlimited open time, within which the material is workable so that the bandage can be applied
- possibility of postponing the hardening until the bandage has been applied, i.e. controllable hardening
- short hardening time from the moment when the hardening is initiated
- handiness and practical usability of the lighting appliance
- freedom from inconvenient measures against harmful light
- insignificant heat development in the lighting appliance during the short hardening time

It applies to the bandage material according to the invention that it
- is easy to put on and shape to the desired bandage
- can be trimmed using an ordinary pair of scissors
- can be made so that it will adhere onto itself so that bandages can be stably built of several layers
- even in its 'self-adhesive' ('pressure-sensitive') version still allows joined layers to be separated, adjusted and joined again
- leaves no adhesive residues on scissors or hands
- can be made so that it is safe for the person applying the bandage
- can be made so that it is safe for the person wearing the bandage
- has a good storage stability (according to preliminary experience, at least 6 months)
- can be manufactured from commodity chemicals and materials by a relatively simple method
- can be handled in ordinary lighting without premature hardening

Besides in stationary operating rooms, the orthopedic bandage set according to the invention is sufficiently sturdy and easy to use for employment
- in ambulances
- in war zones
- on sports grounds etc.
- in treatment of animals

In ambulances, the bandage set of the invention may replace the widely used inflatable provisional bandages with great advantage. Such a bandage may cause considerable inconvenience to the injured person, since for various reasons it has to be removed immediately after arrival at the hospital. Thus, it may affect the blood circulation, just as it excludes observation and examination of the part of the body in question and may impede appropriate positioning of it. However, the removal of the provisional bandage from a broken limb causes acute pain, and anaesthetization cannot be undertaken before the removal. These disadvantages are avoided when a bandage is applied at the scene of accident using the bandage set of the invention. Such a bandage does not impede inspection, X-raying or anaesthetization, and it may in certain cases remain as the final bandage.

### Description of the drawings

Fig. 1 shows a plane lighting appliance with embedded light-emitting diodes.
Fig. 2 shows a piece-of-gutter shaped lighting appliance.
Fig. 3A shows an elastic lighting appliance being placed on a part of the body (cross section).
Fig. 3B shows an elastic lighting appliance which has been placed on a part of the body (cross section).
Fig. 4 shows a lighting appliance having two piece-of-gutter shaped sections for the hardening of bandage material on the leg and the foot.

### Detailed description of the invention

### The constituents of the bandage material

As the invention involves a light hardenable bandage material which is part of an orthopedic bandage set, a detailed description of the constituents of the bandage material follows here.

In order to avoid unnecessarily complicated language, usual joint statements involving both singular and plural forms of nouns are occasionally used in what follows, e.g. 'the passive polymer(s)', which is to be understood as 'the passive polymer or the passive polymers', and 'one or more passive polymer(s)', which is to be understood as 'one passive polymer or more than one passive polymer'.

One constituent of the bandage material is the fibrous, porous or particulate material, which may be loose or coherent. In what follows, this constituent is occasionally referred to as 'carrier material'. The terms 'fibrous', 'porous', 'particulate', 'loose' and 'coherent' are to be understood as referring to the constituent in question as a raw material, i.e. before it is brought into contact with the remaining constituents of the bandage material.

A fibrous material is to be understood as a material mainly consisting of elongated bodies such as fibres, e.g. glass fibres, plastic fibres, plant fibres and hair.

A porous material is to be understood as a material of which a cut-out piece is substantially continuously coherent but contains a number of cavities, each having an unsignificant extension in relation to the dimensions of the whole piece of material being considered, and which cavities may be connected with each other.

A particulate material is to be understood as a material which consists mainly of not pronouncedly oblong, relatively small bodies, which may have an extension of e.g. 0.001 - 1 mm, such as powders or granulated materials. Also a ground or crushed fibrous material may be considered a particulate material if the degree of division is sufficiently high.

A loose material is to be understood as a material consisting of non-bound, relatively small bodies, e.g. particulate materials in which the particles are not glued together or the like, or fibres, e.g. 0.1 - 1 mm long, which are not fixed to each other e.g. by means of an adhesive.

A coherent material is to be understood as a material that can be handled e.g. as cut-out pieces or as lengths such as thread, filament, wire, string, yarn, net, tissue, fabric, felt, paper and foam.

It goes without saying that examples can be found of intermediate forms between loose and coherent materials, e.g. a material containing long fibres.

An example of loose fibrous material is a quantity of cotton fibres which are not mutually connected by spinning, weaving, felting or glueing.

Examples of loose particulate material are powder-like fillers such as ground chalk, quartz and kaolin. The word 'filler' is traditionally used as a common term for solid materials in a finely divided form which are mixed with a more or less liquid material, although they may have other functions in the mixture than just replacing a volume of the more or less liquid material, and the word is used in this sense in what follows.

Examples of coherent fibrous material are spun thread, net, woven fabric and felt of glass fibre, cotton or plastic fibre such as fibre made of polyester or polyamide, the coherence of the material being reached by e.g. mutual mechanical locking of the individual fibres and/or by means of applied binders. A further example of coherent fibrous material is ordinary paper.

An example of coherent porous material is plastic foam, e.g. polyurethane foam, in which a substantial part of the foam cells may be mutually connected, thereby forming a so-called open-celled foam capable of absorbing liquids.

Suitable fibrous, porous or particulate materials to form part of the bandage material are e.g. net, felt, loose fibres, open-celled plastic foam and particulate fillers.

Suitable materials for net are e.g. polyamide, polyester, cotton and glass, and suitable types of net are, among others, such types which by choice of a suitable texture have been made stretchable, and which in a slightly stretched state have e.g. 50 - 500 meshes per meter, e.g. 100 - 250 meshes per meter, and which have a yarn thickness of e.g. 0.3 - 3 mm, e.g. 0.5 - 2 mm, e.g. 0.8 - 1.5 mm. Suitable materials for felt or loose fibres are also e.g. polyamide, polyester, cotton and glass.

Suitable lengths of loose fibres are e.g. 0.3 - 30 mm, e.g. 1 - 20 mm, e.g. 2 - 15 mm, e.g. 4 - 10 mm. The fibres in one bandage material may be of uniform or varying length.

Suitable types of open-celled plastic foam are, among others, soft open-celled foam of e.g. polyurethane.

In the bandage material, the fibrous, porous or particulate material has been impregnated with a light hardenable composition including the constituents I and II and possibly III and possibly IV, the fibrous, porous or particulate material hereby in certain cases acting in the finished bandage material as a reinforcement of the hardened composition.

Of the constituents mentioned, one or more ethylenically unsaturated compounds, I, form a principal constituent. An ethylenically unsaturated compound is to be understood as a compound containing at least one non-aromatic carbon-carbon double bond which can be brought to enter into polymerization and/or crosslinking reactions. By a polymerization and/or crosslinking reaction involving the ethylenically unsaturated constituent(s) of the light hardenable composition, the whole of the bandage material can be brought to change from a more or less liquid or pliable state to the solid state. Particularly easily ethylenically unsaturated compounds having at least one terminal carbon-carbon double bond, such as vinyl compounds, are brought to enter into polymerization and/or crosslinking.

As well vinyl compounds as ethylenically unsaturated compounds which are not vinyl compounds may be part of the light hardenable composition.

Suitable ethylenically unsaturated compounds, which are not vinyl compounds, are e.g. unsaturated polyesters made by condensation between polyols and e.g. unsaturated dicarboxylic acids such as maleic acid.

Examples of suitable classes of vinyl compounds are acrylates, methacrylates and vinyl ethers having molecular weights or mean molecular weights in the range 100 - 7000, e.g. 200 - 5000, e.g. 400 - 4000, e.g. 600 - 3000, e.g. 800 - 2500, e.g. 1200 - 1800. Examples of such compounds are numerous in the literature, see for example G. Webster (ed.): Chemistry & Technology of UV & EB Formulation for Coatings, Inks & Paints, vol. II: Prepolymers & Reactive Diluents, Chichester etc., 1997.

Also vinyl compounds of lower molecular weight such as styrene and vinyltoluene are technically usable in certain compositions, but for health reasons are not preferred.

For adjustment of the properties of the light hardenable composition such as its viscosity and reactivity, ethylenically unsaturated compounds of relatively high molecular weight may be used in combination with ethylenically unsaturated compounds of relatively low molecular weight.

Of importance to the stiffness and the strength of the hardened material is the density of carbon-carbon double bonds in the ethylenically unsaturated compounds. This density can be expressed as the molecular weight divided by the number of carbon-carbon double bonds per molecule. The lower this quotient is, the higher the stiffness will generally be. Preferred values of the quotient, which are not limiting to the invention, are 300 or less, e.g. 200 or less.

In this connection, the molecular weight as well as the number of carbon-carbon double bonds per molecule are to be understood as mean values for a given raw material since commodity chemicals are often mixtures of mutually related compounds with varying data.

In the light hardenable composition, the ethylenically unsaturated compound(s) may together constitute 1 - 99,9999 % by weight of the whole composition, e.g. 5 - 95 % by weight, e.g. 10 - 90 % by weight, e.g. 20 - 80 % by weight, e.g. 30 - 70 % by weight, e.g. 40 - 60 % by weight.

Another principal constituent of the light hardenable composition is the constituent II, which is a light activatable polymerization initiator system of the type which, when activated by exposure to light having at least one wavelength in the range 200 - 750 nm, is capable of initiating polymerization and/or crosslinking of at least one of the ethylenically unsaturated compounds of the light hardenable composition.

Thus, it is possible to use polymerization initiator systems which especially are activated within narrower wavelength ranges, e.g. 250 - 650 nm, e.g. 300 - 550 nm, e.g. 350 - 500 nm, e.g. 380 - 450 nm, e.g. 400 - 420 nm.

Such a polymerization initiator system includes at least one component which on exposure to light of the above-mentioned nature can be brought into an excited state, and at least one component, which may be identical with the first mentioned component, and which is capable of utilizing the light energy taken up by the polymerization initiator system so as to open the carbon-carbon double bonds of one or more of the ethylenically unsaturated compounds of the light hardenable composition, hereby initiating polymerization and/or crosslinking of this compound respectively these compounds.

The polymerization initiator system may consist of one or more components.

The polymerization initiator system may consist of a single component, which both is capable of absorbing light and of utilizing the absorbed light energy to attack carbon-carbon double bonds. A polymerization initiator of this type may be supplemented with a sensitizer which absorbs light of e.g. higher wavelengths than the initiator component which is being supplemented, and which sensitizer through its excited state transfers the absorbed light energy to the initiator component being supplemented, which supplemented initiator component subsequently attacks carbon-carbon double bonds. A two-component system of this type can be designed to be effective in a wider wavelength range than the first mentioned polymerization initiator alone. In other examples of polymerization initiator systems including two components, the components are mutually dependent. Moreover, it is possible to use mixtures of initiators each of which can function alone, but which in combination make possible a higher speed of hardening. These matters have been described in detail in the literature, see for example J.V. Crivello & K. Dietliker: Chemistry & Technology of UV & EB Formulation for Coatings, Inks & Paints, vol. III: Photoinitiators for Free Radical Cationic & Anionic Polymerisation, 2nd edition, Chichester etc., 1998.

The light activatable polymerization initiator system can be designed to have the capability of initiating polymerization and/or crosslinking following different mechanisms, e.g. radical polymerization and cationic polymerization.

The percentage at which the polymerization initiator system is contained in the light hardenable composition is adjusted according to such things as the nature of the initiator system, the nature of the light used for the hardening, the nature and percentage of other constituents of the composition, and the desired speed of hardening. The concentration of the polymerization initiator system may be e.g. 0.01 - 10 % by weight, e.g. 0.03 - 5 % by weight, e.g. 0.1 - 2 % by weight, e.g. 0.2 - 1 % by weight.

Examples of compounds that can function as one-component polymerization initiators of the radical forming type, which are capable of initiating polymerization following a radical mechanism, can be found in compound classes such as alpha-hydroxy ketones, alpha-amino ketones, benzophenones, oxime esters, metallocenes, monoacylphosphine oxides and bis-acylphosphine oxides. Numerous examples of polymerization initiator materials have been described in the literature, see for example J.V. Crivello & K. Dietliker: Chemistry & Technology of UV & EB Formulation for Coatings, Inks & Paints, vol. III: Photoinitiators for Free Radical Cationic & Anionic Polymerisation, 2nd edition, Chichester etc., 1998.

Examples of compounds which are active in polymerization initiator systems capable of initiating polymerization according to a cationic mechanism can also be found in several classes of compounds, e.g. among the iodonium salts.

As a further constituent, the light hardenable composition may include one or more passive polymers. 'Passive' is here to be understood as substantially non-reactive on exposure to light in the presence of a polymerization initiator system and therefore not suited to enter into polymerization or crosslinking reactions with itself or with the ethylenically unsaturated compound(s) of the composition. An in this sense passive polymer is thus, inter alia, characterized in not having in its molecular backbone any more than occasionally occurring carbon-carbon double bonds or other bonds which may be attacked by a polymerization initiator system on exposure to light in the wavelength range and exposure time range used for the hardening of the composition. Such a polymer does not undergo substantial changes of its chemical and physical properties such as its viscosity when subjected to the influence mentioned.

As a constituent of the light hardenable composition, the passive polymer can serve such purposes as modifying the viscosity and elasticity of the ethylenically unsaturated compound(s) of the composition, which ethylenically unsaturated compound(s) may thus be chosen quite freely in relation to molecular weight an viscosity.

As the ethylenically unsaturated compounds which are suitable for use in the light hardenable composition are generally adhesive and leave residues on countersurfaces, which are brought into contact with them, the modification with a passive polymer can be utilized to counteract the tendency of the composition to leave residues when touched. Thus, it becomes possible to produce light hardenable orthopedic bandage materials which are adhesive without tending to leave residues on countersurfaces. Such bandage materials can be handled, cut and trimmed without messing up hands and tools, but has sufficient adhesiveness to facilitate the construction of a coherent bandage from several layers of the bandage material. The property 'adhesive without a tendency to leave residues on countersurfaces' is wellknown from e.g. sticking plaster and 'self-adhesive' or 'pressure-sensitive' tape and labels, but the property has not been described in connection with light hardenable materials for orthopedic bandages.

The passive polymer(s) of the light hardenable composition may each have a mean molecular weight of at least 7000, e.g. in the range 7000 - 10,000,000, e.g. 15,000 - 5,000,000, e.g. 30,000 - 3,000,000, e.g. 60,000 - 2,000,000, e.g. 100,000 - 1,500,000, e.g. 150,000 - 1,000,000, e.g. 200,000 - 600,000, e.g. 250,000 - 400,000, and may each be e.g.
- a homopolymer or copolymer of one or more synthetic monomers such as
   - vinyl alcohol and esters hereof, e.g. vinyl acetate
   - (meth)acrylic acid and esters hereof, e.g. methyl (meth)acrylate, or
- a homopolymer or copolymer including one or more naturally occurring monomers such as esters of unsaturated fatty acids,
   or
- a derivative of a homopolymer or copolymer of one or more synthetic and/or naturally occurring polymers such as polyvinyl acetate,
   or
- a chemically modified form of a naturally occurring polymer, e.g. a cellulose ester or a cellulose ether or an analogous derivative of starch, or partly hydrolyzed starch,
   or
- a naturally occurring polymer, possibly in a physically modified form, such as starch, gelatinized starch, dextrin, pectin, lignin, gelatine, chitin or polyisoprene.

A polymer of one or more given monomers is here to be understood as consisting of structural units corresponding to the monomer(s) in question, but not necessarily as having been synthesized from it or them. Thus, e.g. polyvinyl acetate can be synthesized along different avenues, which do not necessarily include the use of vinyl acetate, see for example Hans-Georg Elias: Macromolecules, vol. II, 2nd edition, New York, 1984.

The passive polymer(s) may make up e.g. 0.1 - 99 % by weight, e.g. 0.5 - 90 % by weight, e.g. 2 - 80 % by weight, e.g. 5 - 70 % by weight, e.g. 10 - 60 % by weight, e.g. 20 - 50 % by weight, e.g. 30 - 40 % by weight of the whole of the composition.

The effects mentioned, which the passive polymer(s) can have on the viscoelastic properties of the composition, are mainly attained when the passive polymer(s) can swell with at least one of the ethylenically unsaturated compounds, in particular when the passive polymer(s) and the ethylenically unsaturated compound(s) can form a homogeneous mixture together.

Finally, the light hardenable composition may include auxiliary materials such as antioxidants, polymerization inhibitors, surfactants such as dispersing agents, and colourants and fillers.

The ratio by weight between the fibrous, porous or particulate carrier material and the light hardenable composition, with which it is impregnated, may vary within wide limits.

In bandage materials which include coherent, fibrous or porous materials of organic nature such as a plastic or cotton having a density of about 1 g/cm³, the fibrous or porous material may make up e.g. 5 - 90 % by weight, e.g. 15 - 60 % by weight, e.g. 25 - 40 % by weight of the whole of the bandage material. In bandage materials which include coherent, fibrous or porous materials of inorganic nature such as glass having a density of about 2.7 g/cm³, the fibrous or porous material may make up e.g. 5 - 95 % by weight, e.g. 15 - 70 % by weight, e.g. 25 - 50 % by weight of the whole of the bandage material.

In bandage materials which include loose fibrous, porous or particulate material, this may make up e.g. 0.5 -95 % by weight, e.g. 2 - 90 % by weight, e.g. 5 - 80 % by weight, e.g. 10 - 70 % by weight, e.g. 20 - 60 % by weight, e.g. 30 - 50 % by weight of the whole of the bandage material.

In its 4th aspect, the invention relates, to a light hardenable orthopedic bandage material including at least one loose or coherent material, which is fibrous, porous or particulate, and which has been impregnated with a light hardenable composition including the constituents I, II and III and possibly IV, where
I includes at least one ethylenically unsaturated compound of vinyl type such as acrylate, methacrylate or vinyl ether, the ethylenically unsaturated compound(s) of vinyl type each having molecular weight in the range 100 - 7000, and the ethylenically unsaturated compound(s) of vinyl type making up 5 - 95 % by weight of the whole of the composition, and where
II includes at least one polymerization initiator material of radical forming type, and where
III includes one or more passive polymers each having a mean molecular weight of at least 7,000, e.g. 7,000 - 10,000,000, said passive polymer(s) each being
   - a homopolymer or copolymer of one or more synthetic monomers such as
      - vinyl alcohol and esters hereof, e.g. vinyl acetate
      - (meth)acrylic acid and esters hereof, e.g. methyl (meth)acrylate,
      or
   - a homopolymer or copolymer including one or more naturally occurring monomers such as esters of unsaturated fatty acids,
      or
   - a derivative of a homopolymer or copolymer of one or more synthetic and/or naturally occurring polymers such as polyvinyl acetate,
      or
   - a chemically modified form of a naturally occurring polymer, e.g. a cellulose ester or a cellulose ether or an analogous derivative of starch, or partly hydrolyzed starch,
   - a naturally occurring polymer, possibly in a physically modified form, such as starch, gelatinized starch, dextrin, pectin, lignin, gelatine, chitin or polyisoprene,
   the passive polymer(s) making up 5 - 95 % by weight of the whole of the composition, and where
IV includes one or more additional auxiliary materials such as antioxidants, polymerization inhibitors, surfactants such as dispersing agents, and colourants and fillers, and where the fibrous, porous or particulate material(s) has, respectively have, been chosen from among net, woven textiles, felt, loose fibres, open-celled foam and particulate filler.

### Production of the bandage material

As the invention in its 5th aspect, includes a method for the production of a light hardenable orthopedic bandage here follows a description of the method, which includes
1) obtaining at least one fibrous, porous or particulate carrier material,
2) obtaining a light hardenable compostion in the form of a mixture of the constituents I, II, III and possibly IV, these constituents being as stated in claim 1 or claim 20, and
3) impregnating the fibrous, porous or particulate material(s) obtained in step 1) with the light hardenable composition obtained in step 2).

'Impregnation of the carrier material with the light hardenable composition' is to be taken to mean that the light hardenable composition is brought to settle on and between the fibres or particles of the carrier material or in its cells, but not necessarily to penetrate in between the molecules of the carrier material.

As a fibrous, porous or particulate carrier material, commercially available types of net, felt, open-celled foam and fillers may be used to a great extent. Coherent materials may have to be cut into suitable lengths or pieces prior to the impregnation.

The light hardenable composition is used to impregnate the carrier material according to the method in dissolved, dispersed or molten form.

When it is desired to use the light hardenable composition in dissolved form, the solution is made first. After this, the fibrous, porous or particulate material is impregnated with the solution, and finally as much as possible of the solvent is brought to evaporate. From a technical point of wiev, a number of volatile organic compounds are well suited for use as a solvent, but for occupational health and cost related reasons only a few of those will be preferred, e.g. acetone and methylene chloride. Also, a mixture of two or more chemical compounds may be used as the solvent. The choice of solvent should be made in consideration of the carrier material, which may possibly be non-resistent to certain solvents. The constituents I, II, III and possibly IV may expediently be dissolved separately in the solvent chosen or possibly in different solvents, with subsequent mixing of the partial solutions. In particular, high molecular weight constituents such as passive polymers are most easily brought into mixture with the other constituents if they have first been dissolved separately, whereas easily soluble constituents such as polymerization initiator materials and ethylenically unsaturated compounds of relatively low molecular weight may often be easily dissolved in turn in the same amount of solvent.

The total concentration in the solvent of the constituents of the light hardenable composition is chosen so that the solution gets a convenient viscosity, but as high as possible in order to minimize the consumption of time and energy for the evaporation of the solvent. Thus, the concentration may be in the range of 20 - 70 % by weight.

In certain cases it may be advantageous to work with a moderately warmed solution, as the viscosity of the materials used is normally a decreasing function of the temperature, so that a moderate warming makes it possible to work with a higher concentration of the light hardenable composition in the solvent.

The method for impregnating the fibrous, porous or particulate material is adapted to the properties of this material and to the form in which it is available.

Coherent carrier materials such as net, felt and foam may conveniently be handled as lengths or pieces, which are taken through the solution, respectively dipped in the solution, or which have the solution applied to them, e.g. by spraying or by means of a roll.

It is also possible to make blanks of the carrier material shaped in 3 dimensions, e.g. including one or two piece-of-gutter shaped sections, for use in the production of bandage material blanks designed for specific purposes, e.g. more or less angular blanks for bandages intended to cover the elbow or the ankle joint. Such spatially shaped blanks of the carrier material can be dipped in the solution or have it applied to them by spraying.

Impregantion of loose carrier materials such as short fibres or particulate fillers can be carried out e.g. by mixing the material to be impregnated with the solution of the light hardenable composition. The mixing can be carried out in e.g. a kneading machine, a stirring mixer, a rolling mill designed for mixing, or an extruder.

After impregnation, the material can be laid out in lengths or pieces, or it can be shaped to bandage material blanks using conventional methods for processing and shaping workable substances.

When an extruder is used as the mixing tool with loose carrier materials, impregnation and shaping may in certain cases be carried out in a unified process in which the extruder has been adapted to produce a continuous profile of desired width and thickness of the impregnated carrier material.

Thus, it is also possible to let the extruder produce a relatively thin rod of impregnated carrier material, which, during or after extrusion, is laid out in a net-like structure. Such a laying-out can be carried out e.g. by moving the supporting surface on which the rod is laid out according to a suitable pattern, preferably in two dimensions, or by producing the rod through a movable nozzle which during extrusion is being moved according to a suitable pattern, in a similar way that a cake can be decorated by 'writing' with whipped cream. By laying out an extruded rod in a controlled manner like this, locking of the rods in relation to each other is not reached through braiding, weaving or knotting as in ordinary textile net, but through adhesive joining of the rods at their points of contact, as the light hardenable composition has a certain adhesiveness. Thus, a net-like structure with square meshes can be produced by first laying out a set of parallel rods and then on top of these a second set of rods at right angles to the rods of the first set. The two sets of rods hereby form adhesive bonds with each other at the points of contact, and the whole structure can be handled as a net. In a similar way, a net-like structure having e.g. rhombic meshes can be formed.

After impregnation and possible shaping of the impregnated carrier material, as much as possible of the solvent is brought to evaporate, e.g. by heating, e.g. in a drying tunnel. For drying pieces and blanks of the bandage material, a suction chamber may also be used.

In the paragraphs on dispersions below, the following terms are used. As a dispersion of the light hardenable composition consists of very fine drops or particles of the composition distributed in e.g. water, the drops or particles of the composition are termed the dispersed phase, whereas the water or the phase corresponding to it is termed the continuous phase.

In order to obtain a dispersion, it may be necessary to let the light hardenable composition contain one or more dispersing agents, i.e. surfactants that promote dispersal of the light hardenable composition in the continuous phase.

When it is desired to use the light hardenable composition in dispersed form, a dispersion of the light hardenable composition is made first, using conventional equipment and conventional methods to make the dispersion. The mixing of the constituents of the composition may conveniently be carried out prior to the dispersing operation, using a heating chamber equipped with mixing means in connection with the dispersing equipment.

After this, the carrier material is impregnated with the dispersion in a manner similar to the procedure used with the dissolved form, and finally the continuous phase is brought to evaporate wholly or partly.

When it is desired to use the light hardenable composition in the molten form, the constituents are mixed at a temperature at which the passive polymer or at least one of the passive polymers is plastic or liquid. The mixing may e.g. be carried out in an extruder, prior to which the polymerization initiator materials and any other auxiliary materials may have been dissolved in or mixed with the ethylenically unsaturated compound(s). During heating and mixing, the constituents may, if necessary, be protected against oxidation from the atmosphere by means of a protective gas such as nitrogen.

The impregnation of the carrier material with the molten light hardenable composition can be carried out in a similar manner as described above for the dissolved form. In case of sufficiently low viscosity, the molten composition can be used for both coherent and loose carrier materials, whereas in case of high viscosity it is mainly suited for the impregnation of loose carrier materials.

The impregnation may also be carried out in a closed mould containing dry carrier material, the molten light hardenable composition being e.g. injected into the carrier material. It is thus possible to produce plane pieces of the bandage material, and it is also possible to produce blanks shaped in 3 dimensions, e.g. having one or two piece-of-gutter shaped sections, as mentioned above under the discussion of the dissolved form of the light hardenable composition.

Loose carrier material impregnated with light hardenable composition may be moulded or rolled out with a net-like or perforated structure, which improves the access of the light to all parts of the structure during hardening of the bandage material, and which makes the finished bandage pleasant to wear as compared with a dense, plate-like bandage.

After impregnation and moulding or rolling, the complete material is cooled.

In general production, the light hardenable bandage material can be made either as continuous profiles which are cut into narrower lengths, or it can be made as more or less finished bandage blanks. After the final shaping and/or cutting, the bandage material can be packed lightproof in convenient numbers per package for distribution to the users. For the packing, release paper may be used which is laid between the sheets or pieces of the bandage material or rolled up together with it in order to prevent layers of the bandage material to stick together. The dimensions to which the bandage material is cut may be chosen so as to correspond to the dimensions of the commercially available thermoplastic and chemically hardening substitutes for Plaster of Paris, where sheets and pieces are typically used for the building of splints, whereas narrow lengths, possibly in roll form, are typically used for circular bandages.

The light hardenable bandage material may further be produced as spatially shaped blanks in a number of designs, the form, size and thickness of the material of each design being adapted to a particular application, such as angular arm bandages to cover at the same time the upper arm, the elbow and the forearm, or angular bandages to cover at the same time the leg, the ankle joint and the foot. The thickness of the material may, as a reinforcement, be increased in certain regions of the bandage blank. By using such pre-formed bandage blanks, the work of cutting and building the bandage individually for each patient may be greatly reduced, or possibly be made unnecessary.

The above description of bandage blanks as being spatially shaped applies to the blanks in their undeformed state, but the blanks may have to be deformed in order to allow packing and distribution in a practical way. For the packing of bandage blanks in a deformed state, release paper may be inserted between mutually opposite surfaces of a bandage blank.

Pieces and blanks of the bandage material, which are only intended for use in single layers, i.e. when it is not intended to build the bandage from more than one layer of the bandage material, may be dusted with talcum or the like in order to make them non-adhesive on that side which is not intended to bond to the initially applied padding, cf. Example 4.

The thickness of the finished bandage material in sheets, pieces or blanks may be e.g. 15 mm or less, e.g. 10 mm or less, e.g. 8 mm or less, e.g. 6 mm or less, e.g. 5 mm or less, e.g. 4 mm or less, e.g. 3 mm or less, e.g. 2 mm or less, e.g. 1 mm or less, e.g. 0.5 mm or less.

### The nature of the lighting appliance

As the invention is a lighting appliance as stated in claim 1, a detailed description of the lighting appliance is given below.

In what follows, the term 'circuit' is used of electrically conductive parts of the lighting appliance and of the power supply and wires etc., although these parts do not at every time form a part of a closed electrical circuit carrying an electric current.

The lighting appliance includes one ore more primary light sources attached to or embedded in a shell-like structure, which is to be understood as a body having two principal surfaces, which principal surfaces either have merging edges along the whole of the circumference, or which principal surfaces along one or more sections of the edge of each principal surface are connected by one or more relatively narrow edge surfaces which edge surfaces each may be composed of more than one single surface, the area of the total edge surface being considerably less, e.g. 5 - 100 times less, e.g. 7 - 50 times less, e.g. 10 - 25 times less than the area of each principal surface. The principal surfaces may be plane or curved, and they may be parallel or approximately parallel.

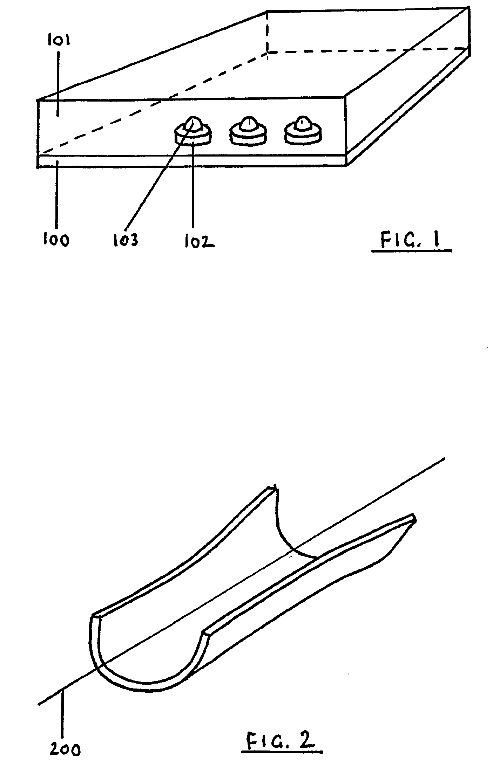

Fig. 1 shows schematically the structure of a plane lighting appliance having embedded light-emitting diodes 102 as primary light sources.

The greatest thickness of the shell-like structure may be in the range 1.5 - 40 mm, e.g. 2 - 30 mm, e.g. 4 - 20 mm, e.g. 6 - 15 mm, e.g. 8 - 12 mm.

The extent of the shell-like structure and thus of the lighting appliance, as measured by means of a flexible tape measure along one principal surface, the tape measure lying closely against the surface, may be in the range 10 - 150 cm, e.g. 15 - 100 cm, e.g. 20 - 70 cm. Depending on the intended use of the lighting appliance, this may have different extent in different directions, so that the lighting appliance appears oblong with a short and a long direction. Assumed that the extents in the two directions are measured by means of tape measures which at their crossing point form a right angle with each other, the extent in the short direction may be e.g. 6 - 60 cm, e.g. 12 - 30 cm, whereas the extent in the long direction may be e.g. 15 - 170 cm, e.g. 25 - 120 cm, e.g. 35 - 90 cm, e.g. 50 - 70 cm. Accordingly, lighting appliances for use for the hardening of bandages for bone fractures may vary in size and in the ratio between the extent in the short direction and the extent in the long direction, the size and the ratio mentioned being adapted to the fracture type, e.g. fracture of the forearm, fracture of the upper arm or fracture of the leg, and to the size of the part of the body in question, which size may, inter alia, depend on whether the patient is a child or an adult.

The shell-like structure in which the primary light sources are embedded or to which they are attached may include solid, foamed, felted or woven material, or possibly a combination of two or more materials.

The materials, of which the shell-like structure is made, include at least one wholly or partly synthetic polymer, e.g. of the classes polysiloxanes, polyolefins, polyacrylates, polyurethanes, polyesters, cellulose esters, and polymers based on epoxides, which classes include both hard and soft materials and both thermoplastic and non-thermoplastic materials.

As the shell-like structure may include hard and/or soft materials, the lighting appliance may be made rigid or flexible.

The shell-like structure may be provided with grooves, indentations or patterns, e.g. which make the structure more easily bendable. Moreover, the structure may be arranged with cavities; for example, the primary light sources may be placed in cavities in order to facilitate a possible replacement in case of failure.

For the formation of the inside of the lighting appliance, materials are preferred which have a good release effect towards the light hardenable bandage material, whereby it is ensured that the surface of the lighting appliance can be easily separated from the surface of the bandage material, both before and after the hardening of the bandage material. Such easily releasing materials are mainly found among materials of low surface tension, including polysiloxanes, polyolefins and fluoropolymers.

In its undeformed state, the lighting appliance may be essentially plane, or it may have one or more curved surfaces, possibly with a varying radius of curvature. 'Undeformed state' is to be understood as the shape that the lighting appliance assumes when the force resulting from the action of mechanical forces on the lighting appliance is zero at any point of the lighting appliance. With not too soft embodiments of the lighting appliance, this state is reached with good approximation when the lighting appliance is left on a plane surface. The undeformed state is reached with excellent approximation when the lighting appliance is placed in a liquid having a density equal to the mean density of the appliance.

In plane embodiments, the lighting appliance may conveniently be flexible, so that it can be wrapped around e.g. an arm or a leg to which bandage material has been applied.

In embodiments having curved surfaces, the lighting appliance may e.g. have a shape approximating that of a piece of gutter, rigid or flexible, so that it is suited for application around a section of an arm or a leg, the lighting appliance hereby being capable of being filled up, wholly or partly, by the bandage material covered section of the part of the body in question.

For the hardening of bandages on arms or legs, where the bandage does not cover the elbow resp. the ankle joint, the lighting appliance may be formed as a gutter-shaped piece, i.e. approximately as a segment of a cylindrical shell, the segment covering e.g. 100 - 180 degrees of the circumference of a cylinder of approximately circular or oval cross section, cf. Fig. 2, where 200 is the axis of the piece of gutter. In such a piece-of-gutter shaped embodiment, the lighting appliance may be rigid or flexible. A rigid lighting appliance may possibly fit less closely to the bandage material than a flexible one, but is still capable of effecting a satisfactory hardening.

A 'piece of gutter' being considered as approximating a segment of a cylindrical shell, in what follows, the angle that a piece-of-gutter shaped lighting appliance in its undeformed state covers of the cylindrical shell is termed the 'enclosing angle'.

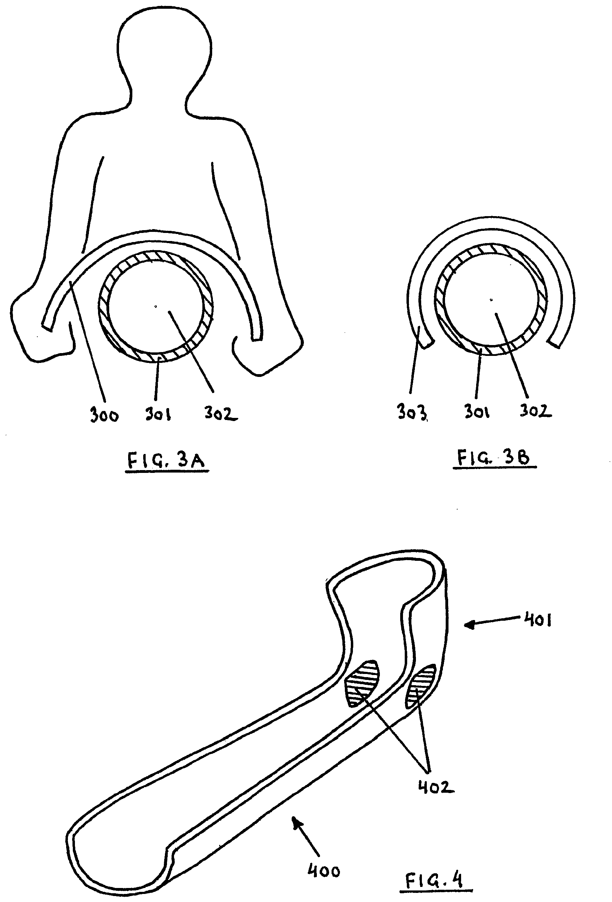

A flexible piece-of-gutter shaped lighting appliance may be made so that it has an enclosing angle of more than 180 degrees, e.g. 190 - 360 degrees, e.g. 240 - 300 degrees. The lighting appliance must then, as shown in Fig. 3A, be opened in order to make it possible to place it on the part of the body in question 302 which has been covered with bandage material 301, but is capable, once it has been placed, of securing itself and remaining in place solely by virtue of the spring-like effect of the lighting appliance 303, i.e. without the use of hands or separate securing means, Fig. 3B.

The lighting appliance may in a further embodiment include two sections each of which are substantially piece-of-gutter shaped, the longitudinal axes of the two sections being non-parallel and forming an angle of e.g. about 90 degrees with each other, so that the lighting appliance is suited for being placed around an elbow or an ankle joint, the one piece-of-gutter shaped section of the lighting appliance hereby being capable of being wholly or partly filled up by a bandage material covered section of the upper arm respectively the leg, the other piece-of-gutter shaped section of the lighting appliance correspondingly being capable of being filled up by a bandage material covered section of the forearm and hand, respectively the foot. Also in this case, the lighting appliance may be made rigid with an enclosing angle not substantially exceeding 180 degrees, or it may be made flexible, allowing the enclosing angle to exceed 180 degrees, in which case the enclosing angle may be e.g. 190 - 360 degrees, e.g. 240 - 300 degrees, whereby the lighting appliance can be self-securing when placed on e.g. a part of the body. In certain cases, e.g. when there is a relatively high friction between the lighting appliance and the material on which it is placed, the lighting appliance can be self-securing when the enclosing angle is merely greater than e.g. 150 degrees. Possibly, the lighting appliance may have both rigid and flexible sections. Fig. 4 shows a lighting appliance having two piece-of-gutter shaped sections 400 and 401 for the hardening of bandage materials on the leg and the foot.

In a similar manner as has been described above, the lighting appliance may be given special designs for use with animals such as dogs and horses, or for non-orthopedic purposes.

For securing the lighting appliance in a bent or closed state around a part of the body, the lighting appliance may, if necessary, be fitted with closing means such as laces, burdock tape, zip fasteners or snap fasteners.

A primary light source is to be understoosd as a device which is capable of transforming electric energy into light, the transformation taking place by the passage of the electric current through e.g. a filament, a gas, a component made of inorganic semiconductor materials or a component made of organic, electroluminescent materials. Thus, the class of primary light sources includes incandescent lamps such as halogen lamps, discharge lamps such as fluorescent lamps, and inorganic and organic light-emitting diodes. In what follows, 'LED' will be used as an abbreviation for 'light-emitting diode' which, if not otherwise specified, is to be understood as referring to LEDs made of inorganic semiconductor materials.

Of the primary light sources included by the lighting appliance, at least one is capable of emitting light having at least one wavelength in the range 200 - 750 nm.

The lighting appliance moreover includes means for supplying electric energy to the primary light sources, including means for connecting the lighting appliance to an external power supply such as sockets for cables or components for inductive or capacitive energy transfer, and internal wiring and possibly current controlling components such as series resistors for supplying each individual primary light source with electric energy.

The electrical wires to the primary light sources may, like the light sources, be embedded in the shell-like structure or attached to it.

Internal electric connections in the lighting appliance can be obtained in several ways, e.g. through the use of separate wires, by means of metal stripes on a structural part which is included in the shell-like structure in which the primary light sources are embedded or to which they are attached, or by means of stripes of conductive paint which is deposited on such a part.

For the prevention of undesired emission of light and thus for the protection of the user's eyes, safety switches may be inserted in the cirquits of the lighting appliance, which safety switches must be switched on before the lighting appliance can be switched on, and which it is not obvious or possible for the user to activate until the lighting appliance has been placed with its inside facing the body to be exposed. The switches may be inserted directly in the power supply cirquits of the primary light sources, or they may be inserted in an auxiliary cirquit which controls the connection and disconnection of the light sources.

Since, for reasons such as the heat generation in the primary light sources, it is not desirable to keep the lighting appliance switched on for longer than the required exposure time, the cirquit may include a timer-controlled switch for accurate control of the exposure time so that the lighting appliance, after having been switched on by the user, is automatically switched off after the set exposure time.

To this may be added an electrically independent signal generating device, which is activated at the same time as the lighting appliance is switched on, and which can be set to produce a light or sound signal by which the operator may check the duration of the exposure.

Moreover, the cirquit may include auxiliary cirquits for the supervision of primary light sources in the lighting appliance, so that an alarm is given if a light source or series of light sources fails. In a simple form, the surveillance may just consist in registrating if a light source or series of light sources is drawing a current. Since a defective primary light source may sometimes draw a current without emitting light, a more elaborate supervision of the individual light sources may be established consisting of registration of both the current through and the voltage drop across the light source and possibly of its temperature or, by means of a photo cell, of the very emission of light.

Of great importance to the capability of the lighting appliance to effect a rapid hardening in a light hardenable orthopedic bandage material is the mean irradiated optical power per unit area, which can be calculated as the sum of the optical powers of the primary light sources included in the lighting appliance divided by the area of the lighting appliance. The lighting appliance may appropriately have an irradiated optical power per unit area of at least 5 W/m², e.g. at least 10 W/m², e.g. at least 15 W/m², e.g. at least 25 W/m², e.g. at least 50 W/m², e.g. at least 75 W/m², e.g. at least 100 W/m², e.g. at least 125 W/m², e.g. at least 150 W/m².

The irradiated optical power per unit area of the lighting appliance depends mainly on two circumstances, i.e. partly on the nature and the light intensity of each of the primary light sources, and partly on the density with which the primary light sources are placed in or on the shell-like structure of the lighting appliance.

The density with which the primary light sources are placed in or on the shell-like structure of the lighting appliance may vary from region to region of the lighting appliance, so that a particularly high optical power can be irradiated from some of its regions as compared with other of its regions. Such an arrangement of the primary light sources may be appropriate in the application of certain types of bandage, e.g. the approximately angular bandages intended to cover the elbow or the ankle joint, where the bandage material near the tip of the angle is laid with double number of layers for the sake of the strength of the finished bandage. In Fig. 4, 402 are regions with a particularly high density of primary light sources. Hereby it is ensured that all material of the bandage can be hardened using the same short exposure time, even though the doubly laid regions of the bandage material require a higher optical power than the singly laid regions for a given hardening time.

Surprisingly, it has become apparent that the primary light sources may, without any drawbacks, be placed at a relatively great mutual distance in the lighting appliance even though the emitted optical power, as measured at points on or near the surface of the lighting appliance, will hereby vary considerably, e.g. by a factor of 5 or more. Thus, for example, if LEDs are used which have a peak wavelength in the range e.g. 380 - 420 nm and an emitted optical power per diode of e.g. 250 mW or more, attainment of hardening times of 1 minute or less with bandage materials according to the 1st and 4th aspect of the invention only requires a density of LEDs in the lighting appliance of e.g. 600 - 750 units per square meter. However, lighting appliances having weaker but possibly more densely placed primary light sources are also included in the invention.

In cases where the primary light sources included in the lighting appliance are embedded in the shell-like structure, the latter must, at least partly, be made of materials which are translucent to the light of the primary light sources.

In what follows, a distinction is made between the two principal surfaces of the lighting appliance, so that 'inside' designates the surface through which the chief part of the optical power is emitted and which has to face the bandage material, whereas 'outside' designates the opposite surface which turns away from the bandage when the lighting appliance has been placed on or folded around the bandage.

As a means of efficient utilization of the light emitted by the primary light sources, light-directing elements or constituents may be embedded in the shell-like structure.

Thus, there may be embedded in the shell-like structure a light-reflecting layer, so that this layer in the lighting appliance is located between the outside of the lighting appliance and the primary light sources. A particularly high degree of light reflection is reached with a metallic layer, which may e.g. be obtained by embedding in the shell-like structure a metal foil or pieces of metal foil or a metallized plastic film or pieces of such a film, or by using small metal flakes as a filler in a layer of the shell-like structure. A high degree of light reflection is reached by using a particulate filler having no considerable light absorption in the wavelength range utilized in the photochemical reactions that one wishes to effect by means of the lighting appliance, the filler being used in a layer of the shell-like structure. Suitable fillers are e.g. magnesium carbonate, calcium carbonate, calcium sulphate, barium sulphate, aluminum oxide, kaolin and talcum.

By use of small metal flakes or a particulate filler, a flexible lighting appliance wil be more easily bendable than it will by use of a continuous foil or film as a light-reflecting layer.

The materials in those parts of the lighting appliance through which the light from the primary light sources has to pass are chosen so that they have sufficient translucency in the wavelength range utilized in the photochemical reactions that one wishes to effect by means of the lighting appliance. The materials may be clear or they may be non-clear and thus light-diffusing, and in the shell-like structure there may be embedded light-directing elements such as lenses, lattices and mirrors. Suitable are e.g. such materials that are made on the basis of polysiloxanes, polyolefins, polyacrylates, polyurethanes and epoxide based polymers.

For the purpose of counteracting undesirable emission of light to the surroundings from the rim of the lighting appliance, a shielding may be placed along the edge.

### Production of the lighting appliance

As the invention in its 3rd aspect relates to a method for producing a lighting appliance as included in the 1st and 2nd aspect, here follows a description of the production of such a lighting appliance.

The building of the shell-like structure, including embedding or attaching the primary light sources, can be carried out in several ways. Thus, the structure can be made by casting or spraying substances which may be drying or hardening and which may be foamed or foaming, or by shaping semimanufactures such as sheet material, film or textiles, using suitable joining methods such as glueing, welding or sewing.

In connection with the 3rd aspect of the invention, it has been realized that the forming of the shell-like structure and the embedment of the primary light sources herein or the attachment of them hereto may be carried out in one concerted operation in which a thermoplastic or a chemically hardening casting material is brought to immobilize and possibly surround completely the primary light sources, these having been brought into the right positions prior to, at the same time as or after introduction of casting material into the mould. Thus, it is possible to arrange a temporally condensed production process, but methods having longer periods between the placing of the primary light sources and the introduction of the casting material are also included in the invention. Using a suitable arrangement of the electrical wiring in the lighting appliance, it is even possible to produce the lighting appliance in lengths, which can eventually be cut to the desired dimensions.

The invention moreover includes lighting appliances in which the shell-like structure, in which the primary light sources are embedded or to which they are attached, is made by going through several separate steps, i.e. a supporting layer can be made in an early step, which is then followed by placing and embedment or attachment of the primary light sources by going through one or more later steps. Thus, the materials from which the shell-like structure is to be made may be taken through one or more steps of shaping, i.e. including moulding, casting, cutting or punching, before they are combined with the primary light sources, and also part of the shell-like structure may be formed directly around the primary light sources, i.e. by moulding or casting.

In accordance with the above, a method for producing the lighting appliance according to the 3rd aspect of the invention may include the following steps :
- obtaining a mould, which can be switched between a closed and an open state, so that the mould in its closed state essentially encloses a cavity which defines the two principal surfaces and the whole of the edge surface of the desired lighting appliance
- placing the mould in its open state and applying a light-reflecting layer on that mould surface which corresponds to the outside of the lighting appliance
- obtaining in the mould an electrical circuit including one or more primary light sources and connecting means
- closing the mould and filling it up with a casting material including at least one wholly or partly synthetic polymer and/or at least one precursor material of at least one such polymer
- awaiting the solidification or hardening of the casting material
- opening the mould and removing the finished lighting appliance The mould can be made of a rigid material such as metal or hard plastic, e.g. glass fibre reinforced polyester, or of a more

flexible material such as polyethylene, possibly of an elastic material such as 'silicone rubber'. For moulding or casting plane embodiments of the lighting appliance, even plane glass plate may be used as material for the mould. With certain casting materials it is possible to use an open-faced, tray-like mould into which casting material is poured, i.e. in these cases the mould need not have two or more movable parts which can enclose a cavity, see Example 3.

The electrical circuit of the lighting appliance, which includes primary light sources and wire connections to these and possibly sockets or components for inductive or capacitive energy transfer, can be obtained in the mould in several ways, e.g. by connecting the components of the circuit to each other outside the mould, e.g. by soldering, and then bringing them into the mould, or by bringing the components into the mould first, e.g. onto the initially obtained, light-reflecting layer which, depending on the binder used, may still be tacky and thus provide securing of the components, and then connecting them to each other, e.g. by soldering.

### Preferred embodiments etc.

As primary light sources of the lighting appliance, LEDs are preferred, either unembedded dies or chips or finished LEDs, possibly with built-in series resistors or other current-controlling circuits. Indeed, ultraviolet and even blue LEDs have proved surprisingly efficient in effecting hardening in light hardenable materials, particularly in such materials which include polymerization initiator systems of the radical-forming type, no matter if these have their absorption maxima far, e.g. 80 - 100 nm, from the emission maxima of the LEDs and if they have only a weak absorption in the wavelength region of the emission maxima of the LEDs.

Of the possible ways in which the LEDs can be placed in the shell-like structure, a surrounding embedment in casting material is preferred rather than a more superficial attachment since a surrounding embedment protects the LEDs and permits a good diffusion of the light from each LED.

In known lighting appliances with LEDs, cooling is necessary because of the heat development in the LEDs. In the lighting appliance according to the invention, however, the rate at which heat can be dissipated from the LEDs is rather unimportant as the temperature does not reach a very high level during the short exposure time, and as the lighting appliance in ordinary use has ample time to give off heat to the surroundings between the exposures.

The low number of LEDs per lighting appliance permits electric connection of the LEDs using a relatively simple wiring and few auxiliary components, even when standard LEDs without built-in series resistors are used, and it facilitates avoidance of high voltage and consequential insulation requirements.

In the lighting appliance according to the invention, it has thus appeared convenient to use a mixed series/parallel connection, in which the diodes of a lighting appliance are arranged in sets, the LEDs in each set being connected in series, and the sets being connected in parallel with each other. However, other types of connection including pure series connection and pure parallel connection are also included in the invention.

In a preferred embodiment of the invention, use is made of a source of direct current, which has built together with it a number of, possibly adjustable, series resistors, each connected to the one terminal of the current source, a wire being drawn from each resistor to a set of mutually series connected LEDs of the lighting appliance, and a common wire being drawn from these sets back to the other terminal of the current source.

In another preferred embodiment, use is made of a direct current source from which two wires are drawn to the lighting appliance where they are connected to parallel connected sets of mutually series connected LEDs, each such set being series connected to a, possibly adjustable, ohmic resistor built together with or built into the lighting appliance.

In a variant of this embodiment, the series resistors are omitted, use being made of calibrated LEDs which are put together in series in such a way that all of the series, when these are parallel connected to each other, draw equal currents, and, if necessary, a series resistor or other current-controlling circuit common to the series being built together with the direct current source.

As it is wellknown that the optical output of LEDs increase with the current, even when this is made higher than the maximum current recommended by the manufacturer, it is possible to operate the LEDs of the lighting appliance of the invention at an increased current and hereby reach a reduction of the exposure time for a bandage relatively to what can otherwise be reached. The also wellknown reduction of the life of the LEDs when these are operated at excess current is unimportant in the lighting appliance according to the invention, since this is only switched on for very short periods, so that the LEDs are not continuedly being subjected to excess current and are not superheated. Also pulsed operation may be used whereby the exposure time can be further reduced.

The lighting appliance may, according to the 6th aspect of the invention, be a part of an arrangement including a power supply for supplying the primary light sources of the lighting appliance with electric energy. The same arrangement may include cables and other connecting components to connect the power supply to the connecting means of the lighting appliance.

The lighting appliance may, according to the 7th aspect of the invention, be applied to the hardening of light hardenable orthopedic bandage material, possibly according to the 1st or the 4th aspect of the invention, see Example 4. The application according to the 7th aspect of the invention may be therapeutic, or it may be non-therapeutic, as light hardenable bandage materials also have a number of possible applications outside the therapeutic field, e.g. as an artistic material and as a structural material for purposes such as the building of prototypes and mock-ups, for the building of exhibitions and for temporary or permanent repairs of various nature.

As apparent from the above description, possible contents of carrier material in the light hardenable bandage material may be within a wide range, e.g. 0.5 - 95 % by weight of the whole of the bandage material. As mentioned, the carrier material may have a reinforcing effect on the light hardenable composition when this has been hardened. This reinforcing effect may, particularly when the carrier material is fibrous or particulate, be quite appreciable. However, recent experiments have shown that in certain cases it is possible to dispense with the carrier material altogether, as it has appeared possible to choose the constituents of the light hardenable composition in such a way that the composition in its hardened state, even without reinforcing material, has sufficient strength as a bandage material, particularly when the material is applied in not too thin layers.

In the case where the orthopedic bandage material of the orthopedic bandage set is not bound to include a carrier material, the invention includes an orthopedic bandage set including
A) a light hardenable bandage material
B) a lighting appliance
where the light hardenable bandage material A includes the constituents I and II, possibly III, possibly IV and possibly V, where the constituents are
I) one or more ethylenically unsaturated compounds,
II) a light activatable polymerization initiator system of the type which, on exposure to light having at least one wavelength in the range 200 - 750 nm, is capable of initiating polymerization of at least one of the ethylenically unsaturated compounds stated under I,
III) one or more passive polymers,
IV) one or more additional auxiliary materials such as antioxidants, polymerization inhibitors, surface active materials such as dispersing agents, and colouring agents and fillers,
V) one or more loose or coherent materials, which are fibrous, porous or particulate, and which have been impregnated with a composition including the constituents I and II and possibly III and possibly IV,
and where the lighting appliance B includes
a shell-like structure including at least one wholly or partly synthetic polymer, which shell-like structure includes two principal surfaces and possibly one or more edge surfaces which connect the principal surfaces, and which shell-like structure has embedded in its interior and/or attached to its one principal surface one or more primary light sources at least one of which primary light sources is capable of emitting light having at least one wavelength in the range 200 - 750 nm,
and where the lighting appliance further includes means for supplying electric energy to the primary light sources, *characterized in* that the shell-like structure has a maximum thickness of 1.5 - 40 mm, and that the shell-like structure preferably has an extent in one direction of 6 - 60 cm and an extent in another direction of 15 - 170 cm,
whereas the invention in its 4th aspect includes
a light hardenable orthopedic bandage material including the constituents I, II and III and possibly IV and possibly V, where
I includes at least one ethylenically unsaturated compound of vinyl type such as acrylate, methacrylate or vinyl ether, the ethylenically unsaturated compound(s) of vinyl type each having molecular weight in the range 100 - 7000, and the ethylenically unsaturated compound(s) of vinyl type making up 5 -95 % by weight of the whole of the composition, and where
II includes at least one polymerization initiator material of radical forming type, and where
III includes one or more passive polymers each having a mean molecular weight of at least 7,000, e.g. 7,000 - 10,000,000, said passive polymer(s) each being
   - a homopolymer or copolymer of one or more synthetic monomers such as
      - vinyl alcohol and esters hereof, e.g. vinyl acetate
      - (meth)acrylic acid and esters hereof, e.g. methyl (meth)acrylate,
      or
   - a homopolymer or copolymer including one or more naturally occurring monomers such as esters of unsaturated fatty acids,
      or
   - a derivative of a homopolymer or copolymer of one or more synthetic and/or naturally occurring polymers such as polyvinyl acetate,
      or
   - a chemically modified form of a naturally occurring polymer, e.g. a cellulose ester or a cellulose ether or an analogous derivative of starch, or partly hydrolyzed starch,
      or
   - a naturally occurring polymer, possibly in a physically modified form, such as starch, gelatinized starch, dextrin, pectin, lignin, gelatine, chitin or polyisoprene,
   the passive polymer(s) making up 5 - 95 % by weight of the whole of the composition, and where
IV includes one or more further auxiliary materials such as antioxidants, polymerization inhibitors, surfactants such as dispersing agents, and colourants and fillers, and where V includes one or more loose or coherent materials, which are fibrous, porous or particulate, and which have been impregnated with a composition including the constituents I, II and III and possibly IV, and which fibrous, porous or particulate materials have been chosen from among net, woven textiles, felt, loose fibres, open-celled foam and particulate filler.

Also in the case where the 1st and the 4th aspect of the invention have the form just described, the invention may include the further particular technical features, or other particular technical features, which have been described in connection with the orthopedic bandage material in the previous paragraphs of the present description.

### Examples

In the examples, the following abbreviations are used:
TMPTA: trimethylolpropane triacrylate (ethylenically unsaturated compound)
TPGDA: tripropyleneglycol triacrylate (ethylenically unsaturated compound)
BDMB: 2-benzyl-2-(dimethylamino)-1-[4-(4-morpholinyl)phenyl]-1-butanone (polymerization initiator)
BBT: bis(*η*⁵-2,4-cyclopentadien-1-yl)bis[2,6-difluoro-3-(1H-pyrrol-1-yl)phenyl]titanium (polymerization initiator)
PVA: polyvinyl acetate (passive polymer)

The two polymerization initiators have the following structures:

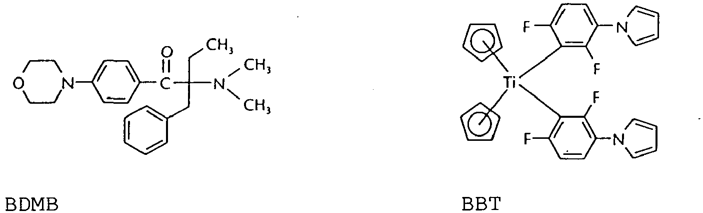

### Example 1. Preparation of a solution of a light hardenable composition.

The solution of the composition comprises

| | |
|---|---|
| TMPTA | 40 g |
| BDMB | 0.9 g |
| PVA of mean molecular weight 245,000 | 50 g |
| Acetone | 65 g |

50 g of PVA of mean molecular weight 250,000 is left to soak in 55 g of acetone at 20 - 30 degrees C for about 2 days after which it will form a clear solution on stirring. 0.9 g of BDMB is dissolved in 10 g of acetone, and this solution is added to the first prepared solution of PVA. Further, 40 g of TMPTA is added, and the solution is stirred until it is homogeneous. The solution is diluted with acetone to a suitable viscosity according to the application. The prepared solution of TMPTA, BDMB and PVA in acetone can be used in making light hardenable bandage materials by impregnation of carrier materials.

### Example 2. Preparation of a light hardenable bandage material.

In this example, the solution of a light hardenable composition prepared in Example 1 is used to impregnate a piece of net, whereby a piece of light hardenable bandage material measuring about 12 x 30 cm is made, which can be used to make an orthopedic bandage (splint) for a fracture in the forearm or the wrist.

As a carrier material is used stretchable, about 2.5 mm thick, knotless net of polyamide having substantially hexagonal masks, where each mask, when the net has been stretched to a degree at which the masks appear as slightly oblong hexagons, is about 8 mm wide. A piece of net is cut so that it, in a slightly stretched state, measures about 12 x 30 cm, and so that the direction of stretchability is parallel to the short side of the piece. The piece is weighed and laid out on a plane support, e.g. a glass plate, and the solution prepared in example 1 is poured over it at a temperature of 18 - 25 degrees C. The piece of net is left to dry in the dark for the evaporation of acetone, possibly in an oven at up to 80 degrees C. After drying, the prepared piece of bandage material is weighed, and the carrier material should then be found to make up about 35 % by weight of the total weight. The piece of bandage material may be placed between sheets of release paper and will keep for several months in a lightproof package at room temperature.

### Example 3. Production of a lighting appliance.

This example concerns the production of a plane, flexible lighting appliance measuring 15 x 35 cm and having blue LEDs as primary light sources.

The LEDs have a peak wavelength of 409 nm with a half width of 14 nm, an emitted optical power 260 mW at 350 mA and an irradiation angle 2*θ*_{½} of 130 degrees, these data being understood as average values for the set of diodes used and as applying to the diodes before the modification of the diodes described below. For the diodes, a maximum current of 400 mA is recommended. The LEDs are embedded in the shell-like structure of the lighting appliance.

As a polymer material for the formation of the shell-like structure, use is made of a translucent, room temperature vulcanizing, 2-component 'silicone rubber' intended for use as an embedding material for electronic components, which in its hardened state has a hardness of 15 - 25 Shore A.

As a mould, use is made of a plane glass plate measuring about 20 x 40 cm with raised edges made by attaching strips of wood or the like along the edges of the plate, e.g. by means of pressure-sensitive tape. As a guidance for the subsequent placement of the LEDs, the pattern according to which the LEDs have to be placed is drawn on the underside of the plate, the LEDs being distributed evenly over the surface of the lighting appliance.

The casting of the shell-like structure is carried out in two operations. Initially, a thin, light-reflecting layer is laid out on the glass plate; on this layer the electric circuit including the LEDs is placed, and finally the translucent embedding material is cast around it.

As a support for laying out the light-reflecting layer, the glass plate is fitted with raised edges, 0.8 mm high and 10 - 15 mm wide, so that the area within the edges measures 15 x 35 cm. The light-reflecting layer is made of equal parts by weight of precipitated barium sulphate and the above-mentioned embedding material with added hardener ('catalyst'), the ingredients being mixed thoroughly. The mixture, which is paste-like, is spread out on the glass plate using a bar sliding on the 0.8 mm high edges.

In this example, use is made of LEDs in standard encapsulation 102, as shown in Fig. 1. The height of the LED including the moulded-on lens is 5.5 mm, but in order to minimize the thickness of the lighting appliance, the lens, which is made of a soft material, is cut off, so that the height of the LED is reduced to about 3 mm.

For the lighting appliance are used 35 LEDs, which are arranged in 5 series connected sets of 7 LEDs each, the LEDs in each set being connected by means of thin wire which is soldered on.

The glass plate carrying the light-reflecting layer, which is not fully hardened and still somewhat tacky, is placed ona a table with built-in light, so that the pattern drawn on the underside can be seen through the light-reflecting layer, and the LEDs are placed on the light-reflecting layer at the marked positions, turning their main direction of light emission away from the light-reflecting layer and at a right angle to it, the 10 cables to/from the 5 sets of LEDs being simply drawn out over the edge of the mould.

The height of the raised edges on the glass plate is now increased to 5 - 10 mm, and into the horizontally placed mould is poured an amount of the above-mentioned embedding material with an addition of 0.2 % by weight of precipitated barium sulphate as a light diffusing agent, so that the level of the embedding material is about 1 mm above the cut lenses of the LEDs. After hardening, the lighting appliance is removed from the mould.

The 5 sets of LEDs are now each connected through an adjustable series resistor of e.g. 0 - 47 ohms to a direct current source, which is capable of yielding at least 2 A at at least 30 V. The series resistors are adjusted to their highest resistance, the voltage is increased slowly, and the series resistors are adjusted so that all of the 5 sets of diodes are drawing a current of 400 mA at the same time, which will happen at a voltage drop of about 24 V across each set of diodes. The arrangement consisting of the lighting appliance with adjusted series resistors and the direct current source can now be used for the hardening of light hardenable bandage materials, the lighting appliance simply being switched on by means of the switch on the direct current source.

### Example 4. Application of an orthopedic bandage.

In this example use is made of light hardenable bandage material as prepared in Example 2 to apply a bandage including a splint to the forearm and wrist of a subject.

In accordance with common clinical practice, the arm is first covered with a thin cotton stocking, which is then wound with padding material. The subject, sitting at a table, places his forearm on the table, the hand closed and the back of the hand upwards. A piece of the bandage material made in Example 2 is placed on the padding, so that it covers the lower part of the forearm and the back of the hand up to the knockles. The bandage material is gently pressed so as to follow the shape of the arm and the hand. On top of the first piece of bandage material a new piece is placed, which is gently pressed, so that the light hardenable composition of the two pieces get into good contact. The pieces of bandage material may, if necessary, be trimmed using an ordinary pair of scissors.

Now the lighting appliance, made an connected to a power supply as described in Example 3, is placed over the bandage material. The lighting appliance, which in this example is not fitted with securing means, can just be held in place around the bandage material by the investigator. The lighting appliance is switched on and is kept switched on for 45 seconds, whereby the bandage material hardens. The lighting appliance is removed, and the hardened splint can now be held in place on the arm by winding arm and splint with elastic bandage material of the type commonly used in clinical practice, after which the subject can confirm that the wrist has been immobilized.

### Example 5. Preparation of a solution of a light hardenable composition.

The solution of the composition comprises

| | |
|---|---|
| TMPTA | 30 g |
| BDMB | 0.8 g |
| PVA of mean molecular weight 85,000 | 50 g |
| Acetone | 65 g |

The same procedure is followed as in Example 1 except that PVA having a mean molecular weight of 85,000 is used instead of the type of PVA used in Example 1. The solution prepared can be used in making light hardenable bandage materials by impregnation of carrier materials.

### Example 6. Preparation of a solution of a light hardenable composition.

The solution of the composition comprises

| | |
|---|---|
| TMPTA | 40 g |
| BBT | 0.3 g |
| PVA of mean molecular weight 245,000 | 50 g |
| Acetone | 65 g |

The same procedure is followed as in Example 1 except that BBT is used as the polymerization initiator instead of the initiator used in Example 1. The solution prepared can be used in making light hardenable bandage materials by impregnation of carrier materials.

### Example 7. Preparation of a solution of a light hardenable composition.

The solution of the composition comprises

| | |
|---|---|
| TMPTA | 40 g |
| BDMB | 0.9 g |
| PVA of mean molecular weight 245,000 | 50 g |
| Methylene chloride | 110 g |

The same procedure is followed as in Example 1 except that methylene chloride is used as the solvent instead of acetone, and that the PVA is soaked in 100 g of methylene chloride. The solution prepared can be used in making light hardenable bandage materials by impregnation of carrier materials.

### Example 8. Preparation of a solution of a light hardenable composition.

The solution of the composition comprises

| | |
|---|---|
| TPGDA | 40 g |
| BDMB | 0.9 g |
| PVA of mean molecular weight 245,000 | 50 g |

| | |
|---|---|
| Acetone | 65 g |

The same procedure is followed as in Example 1 except that TPGDA is used instead of TMPTA as the ethylenically unsaturated compound. The solution prepared can be used in making light hardenable bandage materials by impregnation of carrier materials.

### Example 9. Preparation of a light hardenable composition as a molten material and impregnation of a carrier material herewith.

The composition comprises

| | |
|---|---|
| TPGDA | 60 g |
| BDMB | 1.6 g |
| PVA of mean molecular weight 85,000 | 100 g |

As the carrier material is used 20 g of cellulose fibre having an average fibre length of about 2 mm, prepared by division of cotton wool.

TMPTA is weighed in a glass beaker, and BDMB is dissolved in it. (This may take some time, but BDMB may, without any substantial bearing on the verification of the example, be dissolved in a few milliliters of methylene chloride in the beaker before the TMPTA is introduced). To the solution of BDMB in TMPTA is added PVA, and the mixture is heated while being stirred to about 125 degrees C, e.g. on an oil bath or in a thermostatically controlled, electrical heating jacket. When the mixture has become homogeneous, the cellulose fibres are added, and the substance is stirred until it has become smooth. The impregnated cellulose fibres can be used for the production of light hardenable bandage materials.

### Example 10. Preparation of a light hardenable bandage material.

In this example, the substance consisting of cellulose fibres impregnated with a light hardenable composition, which was made in Example 9, is used for the preparation of a light hardenable bandage material.

The bandage material is made by moulding the above-mentioned substance as an open, net-like structure. For the moulding is used a mould consisting of a plate of semi-rigid 'silicone rubber', about 10 mm thick and measuring about 15 x 35 cm, and bearing a regular lozenge pattern formed by two sets of 3 mm wide and 3 - 4 mm deep grooves, the grooves in one set being parallel to each other, the distance between neighbouring grooves in one set being 10 - 12 mm, and the two sets of grooves intersecting each other at an angle of about 45 degrees. A mould like this can be made by means of a negative mould milled out of metal or plastic from which a cast is taken using a 2-component 'silicone rubber' of ordinary type.

To mould the cellulose fibres impregnated with the light hardenable composition, the mould is placed on a plane plate of e.g. glass or metal and heated together with the latter in an oven to about 125 degrees C. The substance, which has been heated to a suitable consistency, is spread over the mould, so that the grooves are filled, and surplus material is scraped off using a bar. After cooling, the net-like piece of bandage material is removed from the mould. The piece of bandage material may be placed between sheets of release paper and will keep for several months in a lightproof package at room temperature.

### Example 11. Application of an orthopedic bandage

In this example an orthopedic bandage including a splint is applied to the forearm and wrist of a subject, using a light hardenable bandage material such as the material prepared in Example 10.

The same procedure is followed as in Example 4 except that use is made of a single layer of the material prepared in Example 10.

When the bandage has been finished, the subject can, as in Example 4, confirm that the wrist has been immobilized.

### Example 12. Production of a lighting appliance.

In this example, a piece-of-gutter shaped, flexible lighting appliance is produced, which has an extent of 15 x 35 cm when measured using flexible tape measures as described in the detailed description above, the 35 cm being the length of the piece of gutter.

The same materials and components are used as in Example 3, and as in Example 3, the LEDs are embedded in the shell-like structure of the lighting appliance.

The mould is made up of two pieces shaped like segments of cylindrical shells and made of 5 mm clear PMMA (polymethyl methacrylate) sheet, the one shell having an interior radius of 6 cm and designated in what follows as the outer part of the mould, the other shell having an exterior radius of 5.4 cm and designated in what follows as the inner part of the mould, and so that the two parts of the mould can be placed one behind the other with a gap of 6 mm between them. The plates have an extent, as measured along the circumference of the cylindrical shells, of, respectively, 17 cm for the outer part and 15.3 cm for the inner part, and they have a length of 37 cm. The cylindrically shaped pieces of PMMA sheet can be made by softening plane pieces of sheet in an oven followed by folding over e.g. wooden cylinders, which have been turned to the desired radii.

The first step of the casting of the shell-like structure of the lighting appliance is the application of a light-reflecting layer to the inside of the outer part of the mould. For this purpose, the outer part of the mould is fitted with raised edges 1 mm high and about 10 mm wide along the edges, and the light-reflecting layer is applied in a similar way as in Example 3 after which the raised edges are removed.

The 35 LEDs are connected in 5 sets as in Example 3 and placed on the light-reflecting layer. To assist in placing the LEDs, a sheet of clear plastic on which the positions of the diodes have been marked may be placed on the outside of the outer part of the mould and then be transilluminated in a similar way as in Example 3. If necessary, the LEDs may be secured with drops of the embedding material before the mould is closed.

To the mould belongs a rubber gasket to be placed between the parts of the mould along their edges. The gasket is cut from 6 mm thick, half-soft rubber sheet, so that it forms a 10 mm wide frame fitting along the edges of the parts of the mould. Alternatively, the gasket may be cast from room temperature vulcanizing 'silicone rubber'. Of the rectangular, frame-shaped gasket, one of the short sides is cut away, so that the complete mould consisting of an outer part, an inner part and a gasket becomes open along its one curved edge.

The outer part of the mould, carrying the light-reflecting layer on which the LEDs have been placed, is now assembled with the inner part and the gasket, all of the 10 wires to/from the LEDs being drawn out through the open end of the mould cavity. The parts of the mould can be held together by means of pressure-sensitive tape or small clamps. The mould is placed in an upright position standing on the closed, curved edge, i.e. with the open end upwards, and the mould is filled with embedding material of the same composition as in Example 3. After the hardening of the embedding material, the mould is opened, and the lighting appliance is removed.

## Claims

1. A lighting appliance for the hardening of an orthopedic bandage material placed on or built up on the body or on a body member in human beings or animals, which lighting appliance includes a shell-like structure including at least one wholly or partly synthetic polymer, which shell-like structure includes two principal surfaces and possibly one or more edge surfaces, which connect the principal surfaces, and which shell-like structure has embedded in its interior and/or attached to its one principal surface one or more primary light sources, of which primary light sources at least one is capable of emitting light having at least one wavelength in the range 200 - 750 nm,
and which lighting appliance further includes means for supplying electric energy to the primary light sources,
**characterized in that** the shell-like structure has a maximum thickness of 1.5 - 40 mm and **in that** the shell-like structure has a shape approximating that of a piece of gutter or the shell-like structure is flexible, so that it is suited for application around a section of an arm or a leg, and that the shell-like structure preferably has an extent in one direction of 6 - 60 cm and an extent in another direction of 15 - 170 cm.

2. A lighting appliance according to claim 1, where at least one, preferably all, of the primary light sources of the lighting appliance are chosen from among LEDs and halogen lamps.

3. A lighting appliance according to claim 1 or 2, where the shell-like structure of the lighting appliance has substantially the shape of a piece of gutter when the lighting appliance is undeformed, so that the lighting appliance is suited for being placed on or around an arm or a leg, the lighting appliance hereby being capable of being filled up by the arm or the leg, respectively; and/or where the shell-like structure of the lighting appliance includes two sections, each of which two sections is substantially piece-of-gutter shaped when the lighting appliance is undeformed, and the longitudinal axes of which two sections are non-parallel, possibly forming an angle of e.g. about 90 degrees with each other, so that the lighting appliance is suited for being placed on or around an elbow or an ankle joint, one section hereby being capable of being filled up by the upper arm or the leg, respectively, the other section hereby being capable of being filled up by the forearm or the foot, respectively.

4. A lighting appliance according to claim 3 , where at least one piece-of-gutter shaped section of the lighting appliance has at least locally an enclosing angle of at least 150 degrees, and where the lighting appliance is at least partly elastically bendable, so that the section or sections of the lighting appliance which have an enclosing angle of at least 150 degrees can be opened and brought to at least partly enclose a body, and so that said sections after having been placed on the body are capable of securing the lighting appliance in place on the body.

5. A lighting appliance according to any of the claims 1 - 4, where at least one primary light source of the lighting appliance is capable of emitting light having at least one wavelength in the range 300 - 700 nm, e.g. 330 - 600 nm, e.g. 360 - 550 nm, e.g. 390 - 500 nm, e.g. 420 - 470 nm.

6. A lighting appliance according to any of the claims 1 - 5, where the lighting appliance is capable of irradiating an optical effect per unit area of at least 5 W/m², e.g. at least 10 W/m², e.g. at least 15 W/m², e.g. at least 25 W/m², e.g. at least 50 W/m², e.g. at least 75 W/m², e.g. at least 100 W/m², e.g. at least 125 W/m², e.g. at least 150 W/m²,

7. A lighting appliance according to any of the claims 1 - 6, where the density of primary light sources of the lighting appliance varies from one region of the principal surface of the lighting appliance to another region of the principal surface of the lighting appliance.

8. A lighting appliance according to any of the claims 1 - 7, where the lighting appliance emits the chief part of the light through its one principal surface, there being arranged between the primary light sources and the other principal surface a light-reflecting layer, which reduces the transmission of light through this other principal surface; and/or where the wholly or partly synthetic polymer or polymers of the shell-like structure includes a light-diffusing agent which includes e.g. finely divided magnesium carbonate, calcium carbonate, calcium sulphate, barium sulphate, aluminum oxide, kaolin or talcum.

9. An orthopedic bandage set including
A) a light hardenable bandage material
B) a lighting appliance according to any of the preceding claims
where the light hardenable bandage material A includes at least one loose or coherent material, which is fibrous, porous or particulate, and which has been impregnated with the light hardenable composition including the constituents I and II, possibly III and possibly IV:
I) one or more ethylenically unsaturated compounds,
II) a light activatable polymerization initiator system of the type which, on exposure to light having at least one wavelength in the range 200 - 750 nm, is capable of initiating polymerization of at least one of the ethylenically unsaturated compounds stated under I,
III) one or more passive polymers,
IV) one or more additional auxiliary materials such as antioxidants, polymerization inhibitors, surface active materials such as dispersing agents, and colouring agents and fillers.

10. An orthopedic bandage set according to claim 9, where at least one ethylenically unsaturated compound of the light hardenable composition is of the vinyl type such as acrylate, methacrylate or vinyl ether, where the ethylenically unsaturated compound(s) of the vinyl type each has a molecular weight in the range 100 - 7000, and where the ethylenically unsaturated compound(s) make up 5 - 95 % by weight of the whole of the composition.

11. An orthopedic bandage set according to claim 9 or claim 10, where the light activatable polymerization initiator system of the light hardenable composition includes at least one initiator material of the radical forming type such as
2-benzyl-2-(dimethylamino)-1-[4-(4-morpholinyl)phenyl]-1-butanone or
bis(η5-2,4-cyclopentadien-1-yl)bis[2,6-difluoro-3-(1H-pyrrol-1-yl)phenyl]titanium or
α,α-dimethoxy-α-phenylacetophenone or
an acylphosphine oxide, possibly a bis-acylphosphine oxide

12. An orthopedic bandage set according to any of the claims 9-11, where the light hardenable composition includes one or more passive polymers, each having a mean molecular weight of at least 7,000, e.g. 7,000 - 10,000,000, the passive polymer(s) each being
- a homopolymer or copolymer of one or more synthetic monomers such as
- vinyl alcohol and esters hereof, e.g. vinyl acetate
- (meth)acrylic acid and esters hereof, e.g. methyl (meth)acrylate,
or
- a homopolymer or copolymer including one or more naturally occurring monomers such as esters of unsaturated fatty acids, or
- a derivative of a homopolymer or copolymer of one or more synthetic and/or naturally occurring polymers such as polyvinyl acetate,
or
- a chemically modified form of a naturally occurring polymer, e.g. a cellulose ester or a cellulose ether or an analogous derivative of starch, or partly hydrolyzed starch,
or
- a naturally occurring polymer, possibly in a physically modified form, such as starch, gelatinized starch, dextrin, pectin, lignin, gelatine, chitin or polyisoprene,
the passive polymers making up 5 - 95 % by weight of the whole of the composition.

13. An orthopedic bandage set according to any of the claims 9 - 12, where the fibrous, porous or particulate material of the bandage material has been chosen from among net, woven fabric, felt, loose fibres, open-celled foam or particulate filler.

14. Use of a lighting appliance as stated in any of the claims 9 - 13 and/or according to any of the claims 1 - 8 for hardening a light hardenable orthopedic bandage material where the bandage material may be as stated in any of the claims 9 - 13; with the proviso that said application is non-therapeutic.

15. Use according to claim 14, where the hardened material is produced by exposure to light from the lighting appliance for a period of 120 seconds or less, e.g. 90 seconds or less, e.g. 60 seconds or less, e.g. 45 seconds or less, e.g. 30 seconds or less, e.g. 15 seconds or less, e.g. 10 seconds or less, e.g. 5 seconds or less, e.g. 2 seconds or less, e.g. 1 second or less.

## Patentansprüche

1. Beleuchtungsvorrichtung zum Härten eines orthopädischen Bandagenmaterials, das auf dem Körper oder auf einem Körperteil bei Menschen oder Tieren platziert oder darauf aufgetragen worden ist, welche Beleuchtungsvorrichtung eine hüllenartige Struktur, welche mindestens ein vollständig oder teilweise synthetisches Polymer umfasst, umfasst, welche hüllenartige Struktur zwei hauptsächliche Oberflächen und gegebenenfalls eine oder mehrere Kantenoberflächen, die die hauptsächlichen Oberflächen verbinden, umfasst und welche hüllenartige Struktur eingebettet in ihr Inneres und/oder angeheftet an eine von deren hauptsächlichen Oberflächen eine oder mehrere primäre Lichtquellen aufweist, wobei von diesen primären Lichtquellen mindestens eine in der Lage ist, Licht mit mindestens einer Wellenlänge im Bereich von 200 - 750 nm zu emittieren,
und welche Beleuchtungsvorrichtung des Weiteren Mittel zum Versorgen der primären Lichtquellen mit elektrischer Energie umfasst,
**dadurch gekennzeichnet, dass** die hüllenartige Struktur eine maximale Dicke von 1,5 - 40 mm aufweist und dass die hüllenartige Struktur eine Gestalt aufweist, die jener eines Rinnenstücks nahekommt, oder die hüllenartige Struktur flexibel ist, so dass sie für eine Anwendung um einen Abschnitt eines Arms oder eines Beins geeignet ist, und dass die hüllenartige Struktur vorzugsweise eine Ausdehnung in einer Richtung von 6 - 60 cm und eine Ausdehnung in einer anderen Richtung von 15 - 170 cm aufweist.

2. Beleuchtungsvorrichtung nach Anspruch 1, wobei mindestens eine, vorzugsweise alle, von den primären Lichtquellen der Beleuchtungsvorrichtung unter LEDs und Halogenlampen ausgewählt ist bzw. sind.

3. Beleuchtungsvorrichtung nach Anspruch 1 oder 2, wobei die hüllenartige Struktur der Beleuchtungsvorrichtung im Wesentlichen die Gestalt eines Rinnenstücks aufweist, wenn die Beleuchtungsvorrichtung unverformt ist, so dass die Beleuchtungsvorrichtung geeignet ist, um auf oder um einen Arm oder ein Bein herum platziert zu werden, wobei die Beleuchtungsvorrichtung dadurch in der Lage ist, durch den Arm bzw. das Bein aufgefüllt zu werden; und/oder wobei die hüllen artige Struktur der Beleuchtungsvorrichtung zwei Abschnitte umfasst, jeder der beiden Abschnitte im Wesentlichen wie ein Stück Rinne geformt ist, wenn die Beleuchtungsvorrichtung unverformt ist, und die Längsachsen von diesen zwei Abschnitten nicht-parallel sind, wobei sie gegebenenfalls einen Winkel von z.B. ungefähr 90 Grad miteinander bilden, so dass die Beleuchtungsvorrichtung geeignet ist, um auf oder um einen Ellenbogen oder ein Knöchelgelenk herum platziert zu werden, wobei ein Abschnitt dadurch in der Lage ist, durch den Oberarm bzw. das Bein aufgefüllt zu werden, wobei der andere Abschnitt dadurch in der Lage ist, durch den Unterarm bzw. den Fuß aufgefüllt zu werden.

4. Beleuchtungsvorrichtung nach Anspruch 3, wobei mindestens ein die Gestalt eines Rinnenstücks aufweisender Abschnitt der Beleuchtungsvorrichtung mindestens lokal einen einschließenden Winkel von mindestens 150 Grad aufweist und wobei die Beleuchtungsvorrichtung mindestens teilweise elastisch biegbar ist, so dass der Abschnitt oder die Abschnitte der Beleuchtungsvorrichtung, die einen einschließenden Winkel von mindestens 150 Grad aufweisen, geöffnet und dazu gebracht werden können, mindestens teilweise einen Körper einzuschließen, und so dass die Abschnitte, nachdem sie auf dem Körper platziert worden sind, in der Lage sind, die Beleuchtungsvorrichtung auf dem Körper an Ort und Stelle zu fixieren.

5. Beleuchtungsvorrichtung nach einem der Ansprüche 1-4, wobei mindestens eine primäre Lichtquelle der Beleuchtungsvorrichtung in der Lage ist, Licht mit mindestens einer Wellenlänge im Bereich von 300 - 700 nm, z.B. 330 - 600 nm, z.B. 360 - 550 nm, z.B. 390 - 500 nm, z.B. 420 - 470 nm zu emittieren.

6. Beleuchtungsvorrichtung nach einem der Ansprüche 1-5, wobei die Beleuchtungsvorrichtung in der Lage ist, eine optische Leistung pro Einheitsfläche von mindestens 5 W/m², z.B. mindestens 10 W/m², z.B. mindestens 15 W/m², z.B. mindestens 25 W/m², z.B. mindestens 50 W/m², z.B. mindestens 75 W/m², z.B. mindestens 100 W/m², z.B. mindestens 125 W/m², z.B. mindestens 150 W/m² auszustrahlen.

7. Beleuchtungsvorrichtung nach einem der Ansprüche 1-6, wobei die Dichte von primären Lichtquellen der Beleuchtungsvorrichtung von einer Region der hauptsächlichen Oberfläche der Beleuchtungsvorrichtung zu einer anderen Region der hauptsächlichen Oberfläche der Beleuchtungsvorrichtung variiert.

8. Beleuchtungsvorrichtung nach einem der Ansprüche 1-7, wobei die Beleuchtungsvorrichtung den hauptsächlichen Teil des Lichts durch eine ihrer hauptsächlichen Oberflächen emittiert, wobei zwischen den primären Lichtquellen und der anderen hauptsächlichen Oberfläche eine Licht reflektierende Lage angeordnet ist, welche den Durchlass von Licht durch diese andere hauptsächliche Oberfläche verringert; und/oder wobei das oder die vollständig oder teilweise synthetische(n) Polymer oder Polymere der hüllenartigen Struktur ein lichtdiffundierendes Mittel, welches z.B. fein verteiltes Magnesiumcarbonat, Calciumcarbonat, Calciumsulfat, Bariumsulfat, Aluminiumoxid, Kaolin oder Talkum umfasst, umfasst.

9. Orthopädische Bandagengarnitur, umfassend
A) ein durch Licht härtbares Bandagenmaterial,
B) eine Beleuchtungsvorrichtung nach einem der vorangegangenen Ansprüche,
wobei das durch Licht härtbare Bandagenmaterial A mindestens ein loses oder zusammenhängendes Material, das faserförmig, porös oder teilchenförmig ist, und welches mit der durch Licht härtbaren Zusammensetzung umfassend die Bestandteile I und II, gegebenenfalls III und gegebenenfalls IV, imprägniert ist, umfasst;
I) ein oder mehrere ethylenisch ungesättigte Verbindungen,
II) ein durch Licht aktivierbares Polymerisationsinitiatorsystem des Typs, welcher bei Exposition gegenüber Licht mit mindestens einer Wellenlänge im Bereich von 200 - 750 nm in der Lage ist, eine Polymerisation von mindestens einer der unter I angegebenen ethylenisch ungesättigten Verbindungen zu starten,
III) ein oder mehrere passive Polymere,
IV) ein oder mehrere zusätzliche Hilfsmaterial(ien), wie Antioxidationsmittel, Polymerisationsinhibitoren, grenzflächenaktive Materialien, wie Dispergiermittel, und Färbemittel und Füllstoffe.

10. Orthopädische Bandagengarnitur nach Anspruch 9, wobei mindestens eine ethylenisch ungesättigte Verbindung der durch Licht härtbaren Zusammensetzung vom Vinyltyp, wie Acrylat, Methacrylat oder Vinylether, ist, wobei die ethylenisch ungesättigte(n) Verbindung(en) vom Vinyltyp jeweils ein Molekulargewicht im Bereich von 100-7000 aufweist bzw. aufweisen und wobei die ethylenisch ungesättigte(n) Verbindung(en) 5 bis 95 Gew.-% der Gesamtheit der Zusammensetzung ausmachen.

11. Orthopädische Bandagengarnitur nach Anspruch 9 oder Anspruch 10, wobei das durch Licht aktivierbare Polymerisationsinitiatorsystem der durch Licht härtbaren Zusammensetzung mindestens ein Initiatormaterial des Radikale bildenden Typs wie
2-Benzyl-2-(dimethylamino)-1-[4-(4-morpholinyl)phenyl]-1-butanon oder
Bis(η5-2,4-cyclopentadien-1-yl)bis[2,6-difluor-3-(1H-pyrrol-1-yl)phenyl]titan oder
α,α-Dimethoxy-α-phenylacetophenon oder
ein Acylphosphinoxid, gegebenenfalls ein Bisacylphosphinoxid
umfasst.

12. Orthopädische Bandagengarnitur nach einem der Ansprüche 9-11, wobei die durch Licht härtbare Zusammensetzung ein oder mehrere passive Polymere umfasst, wobei jedes ein mittleres Molekulargewicht von mindestens 7.000, z.B. 7.000-10.000.000, aufweist, wobei das bzw. die passive(n) Polymer(e) jeweils
- ein Homopolymer oder Copolymer von einem oder mehreren synthetischen Monomeren wie
- Vinylalkohol und Ester davon, z.B. Vinylacetat,
- (Meth)acrylsäure und Ester davon, z.B. Methyl(meth)acrylat,
oder
- ein Homopolymer oder Copolymer umfassend ein oder mehrere in der Natur vorkommende Monomere, wie Ester von ungesättigten Fettsäuren,
oder
- ein Derivat eines Homopolymers oder Copolymers von einem oder mehreren synthetischen und/oder in der Natur vorkommenden Polymeren, wie Polyvinylacetat,
oder
- eine chemisch modifizierte Form eines in der Natur vorkommenden Polymers, z.B. ein Celluloseester oder ein Celluloseether oder ein analoges Derivat von Stärke oder teilweise hydrolysierte Stärke,
oder
- ein in der Natur vorkommendes Polymer, gegebenenfalls in einer physikalisch modifizierten Form, wie Stärke, gelierte Stärke, Dextrin, Pektin, Lignin, Gelatine, Chitin oder Polyisopren,
ist bzw. sind, wobei die passiven Polymere 5 bis 95 Gew.-% der Gesamtheit der Zusammensetzung ausmachen.

13. Orthopädische Bandagengarnitur nach einem der Ansprüche 9-12, wobei das faserförmige, poröse oder teilchenförmige Material des Bandagenmaterials unter Netz, gewobenem Stoff, Filz, losen Fasern, offenporigem Schaumstoff oder teilchenförmigem Füllstoff ausgewählt worden ist.

14. Verwendung einer Beleuchtungsvorrichtung nach einem der Ansprüche 9-13 und/oder nach einem der Ansprüche 1 - 8 zum Härten eines durch Licht härtbaren orthopädischen Bandagenmaterials, wobei das Bandagenmaterial wie in einem der Ansprüche 9 - 13 angegeben sein kann, mit der Maßgabe, dass die Anwendung nicht-therapeutisch ist.

15. Verwendung nach Anspruch 14, wobei das gehärtete Material hergestellt wird durch Exposition gegenüber Licht ausgehend von der Beleuchtungsvorrichtung für eine Zeitspanne von 120 Sekunden oder weniger, z.B. 90 Sekunden oder weniger, z.B. 60 Sekunden oder weniger, z.B. 45 Sekunden oder weniger, z.B. 30 Sekunden oder weniger, z.B. 15 Sekunden oder weniger, z.B. 10 Sekunden oder weniger, z.B. 5 Sekunden oder weniger, z.B. 2 Sekunden oder weniger, z.B. 1 Sekunde oder weniger.

## Revendications

1. Dispositif d'éclairage pour le durcissement d'un matériau de bandage orthopédique placé ou formé sur le corps ou un élément corporel à des êtres humains ou animaux, lequel dispositif d'éclairage comporte une structure de type coque comportant au moins un polymère entièrement ou partiellement synthétique, laquelle structure en forme de coque comporte deux surfaces principales et éventuellement une ou plusieurs surfaces de bordure, qui relient les surfaces principales, et laquelle structure en forme de coque a, intégrée(s) à l'intérieur de celle-ci et/ou fixé à sa surface principale, une ou plusieurs source(s) de lumière(s) primaire(s), et parmi les sources de lumière primaires, au moins l'une est capable d'émettre une lumière ayant au moins une longueur d'ondes dans la plage de 200 à 750 nm,
et lequel dispositif d'éclairage comporte en outre des moyens pour fournir une énergie électrique aux sources de lumière primaires,
**caractérisé en ce que** la structure en forme de coque a une épaisseur maximale de 1,5 à 40 mm et **en ce que** la structure en forme de coque a une forme s'approchant de celle d'un morceau de gouttière ou la structure en forme de coque est flexible de sorte qu'elle est appropriée pour une application autour d'une partie d'un bras ou d'une jambe, et **en ce que** la structure en forme de coque a de préférence une étendue dans une direction de 6 à 60 cm et une étendue dans une autre direction de 15 à 170 cm.

2. Dispositif d'éclairage selon la revendication 1, où au moins une, de préférence, toutes les sources de lumière primaires du dispositif d'éclairage sont choisies parmi les LED et les lampes halogènes.

3. Dispositif d'éclairage selon la revendication 1 ou 2, où la structure en forme de coque du dispositif d'éclairage a substantiellement la forme d'un morceau de gouttière lorsque le dispositif d'éclairage est non déformé de sorte que le dispositif d'éclairage est approprié pour être placé sur ou autour d'un bras ou d'une jambe, le dispositif d'éclairage pouvant ainsi être rempli par le bras ou la jambe, respectivement ; et/ou où la structure en forme de coque du dispositif d'éclairage comporte deux parties, chacune des deux parties étant substantiellement en forme de morceau de gouttière lorsque le dispositif d'éclairage est non déformé, et les axes longitudinaux desdites deux parties sont non parallèles, formant éventuellement un angle par exemple d'environ 90 degrés l'une avec l'autre, de sorte que le dispositif d'éclairage est approprié pour être placé sur ou autour d'un coude ou d'une cheville, une partie pouvant ainsi être remplie par le haut du bras ou de la jambe, respectivement, l'autre partie pouvant ainsi être remplie par l'avant-bras ou le pied, respectivement.

4. Dispositif d'éclairage selon la revendication 3, où au moins une section en forme de morceau de gouttière du dispositif d'éclairage a au moins localement un angle obtus d'au moins 150 degrés, et où le dispositif d'éclairage peut être courbé au moins partiellement de façon élastique de sorte que la partie ou les parties du dispositif d'éclairage qui ont un angle obtus d'au moins 150 degrés peuvent être ouverte(s) et amenées à renfermer au moins partiellement un corps et de sorte que lesdites parties après avoir été placées sur le corps peuvent fixer le dispositif d'éclairage en place sur le corps.

5. Dispositif d'éclairage selon l'une quelconque des revendications 1 à 4, où au moins une source de lumière primaire du dispositif d'éclairage est capable d'émettre une lumière ayant au moins une longueur d'ondes dans la plage de 300 à 700 nm, par exemple, de 330 à 600 nm, par exemple, de 360 à 550 nm, par exemple de 390 à 500 nm, par exemple de 420 à 470 nm.

6. Dispositif d'éclairage selon l'une quelconque des revendications 1 à 5, où le dispositif d'éclairage peut irradier un effet optique par zone unitaire d'au moins 5 W/m², par exemple d'au moins 10 W/m², par exemple d'au moins 15 W/m², par exemple d'au moins 25 W/m², par exemple d'au moins 50 W/m², par exemple d'au moins 75 W/m², par exemple d'au moins 100 W/m², par exemple d'au moins 125 W/m², par exemple d'au moins 150 W/m².

7. Dispositif d'éclairage selon l'une quelconque des revendications 1 à 6, où la densité des sources de lumière primaires du dispositif d'éclairage varie d'une région de la surface principale du dispositif d'éclairage à une autre région de la surface principale du dispositif d'éclairage.

8. Dispositif d'éclairage selon l'une quelconque des revendications 1 à 7, où le dispositif d'éclairage émet la majeure partie de la lumière au travers de sa surface principale, où est agencée entre les sources de lumière primaire et l'autre surface principale, une couche reflétant la lumière, qui réduit la transmission de la lumière au-travers de cette autre surface principale ; et/ou où le polymère ou les polymères entièrement ou partiellement synthétiques de la structure en forme de coque comporte(nt) un agent diffusant de la lumière qui comporte par exemple, finement divisés, du carbonate de magnésium, du carbonate de calcium, du sulfate de calcium, du sulfate de baryum, de l'oxyde d'aluminium, du kaolin ou du talc.

9. Ensemble de bandage orthopédique comportant
A) un matériau de bandage photodurcissable
B) un dispositif d'éclairage selon l'une quelconque des revendications précédentes,
où le matériau de bandage thermodurcissable A comporte au moins un matériau libre ou homogène qui est fibreux, poreux ou particulaire, et qui a été imprégné avec la composition thermodurcissable comportant les constituants I et II, éventuellement III et éventuellement IV ;
I) un ou plusieurs composés éthyléniquement insaturés
II) un système d'initiateur de polymérisation thermo-activable du type qui, lors d'une exposition à la lumière ayant au moins une longueur d'ondes dans la plage de 200 à 750 nm, peut initier une polymérisation d'au moins d'un des composés éthyléniquement insaturés définis au point I,
III) un ou plusieurs polymères passifs,
IV) un ou plusieurs matériaux auxiliaires supplémentaires tels que des antioxydants, des inhibiteurs de polymérisation, des matériaux tensioactifs tels que des agents dispersants, et des agents colorants et agents de charge.

10. Ensemble de bandage orthopédique selon la revendication 9, où au moins un composé éthyléniquement insaturé de la composition photodurcissable est de type vinyle tel qu'un acrylate, méthacrylate ou éther de vinyle, où le/les composé(s) éthyléniquement insaturé(s) de type vinyle ont respectivement un poids moléculaire dans la plage de 100 à 7000, et où le/les composé(s) éthyléniquement insaturé(s) constituent 5 à 95 % en poids de la totalité de la composition.

11. Ensemble de bandage orthopédique selon la revendication 9 ou la revendication 10, où le système initiateur de polymérisation photoactivable de la composition photodurcissable comporte au moins un matériau initiateur du type formant un radical tel que
la 2-benzyl-2-(diméthylamino)-1-[4-(4-morpholinyl)phényl]-1-butanone ou
le bis(η5-2,4-cyclopentadién-1-yl)bis[2,6-difluoro-3-(1H-pyrrol-1-yl)phényl]titane ou
l'α,α-diméthoxy-α-phénylacétophénone ou
un oxyde d'acylphosphine, éventuellement un oxyde de bis-acylphosphine.

12. Ensemble de bandage orthopédique selon l'une quelconque des revendications 9 à 11, où la composition thermodurcissable comporte un ou plusieurs polymères passifs, chacun ayant un poids moléculaire moyen d'au moins 7000, par exemple 7000 à 10 000 000, le/les polymère(s) passif(s) étant respectivement
- un homopolymère ou copolymère d'un ou plusieurs monomères synthétiques tels que
- un alcool vinylique et des esters de celui-ci, par exemple l'acétate de vinyle
- un acide (méth)acrylique et des esters de celui-ci, par exemple le méthyl(méth)acrylate,
ou
- un homopolymère ou copolymère comportant un ou plusieurs monomères présents à l'état naturel tel que des esters d'acides gras insaturés,
ou
- une forme chimiquement modifiée d'un polymère présent à l'état naturel, par exemple, un ester de cellulose ou un éther de cellulose ou un dérivé d'amidon analogue ou un amidon partiellement hydrolysé,
ou
- un polymère présent à l'état naturel, éventuellement sous une forme physiquement modifiée telle que l'amidon, l'amidon gélatinisé, la dextrine, la pectine, la lignine, la gélatine, la chitine ou le polyisoprène, les polymères passifs constituant 5 à 95 % en poids de la totalité de la composition.

13. Ensemble de bandage orthopédique selon l'une quelconque des revendications 9 à 12, où le matériau fibreux, poreux ou particulaire du matériau de bandage a été choisi parmi un filet, un tissu tissé, un feutre, des fibres libres, de la mousse à cellules ouvertes ou un agent de charge particulaire.

14. Utilisation d'un dispositif d'éclairage selon l'une quelconque des revendications 9 à 13 et/ou selon l'une quelconque des revendications 1 à 8, pour le durcissement d'un matériau de bandage orthopédique photodurcissable où le matériau de bandage peut être tel que défini dans l'une quelconque des revendications 9 à 13 ; à condition que ladite application soit non thérapeutique.

15. Utilisation selon la revendication 14, où le matériau durci est produit par exposition à la lumière du dispositif d'éclairage pendant une période de 120 secondes ou moins, par exemple 90 secondes ou moins, par exemple 60 secondes ou moins, par exemple, 45 secondes ou moins, par exemple, 30 secondes ou moins, par exemple, 15 secondes ou moins, par exemple 10 secondes ou moins, par exemple 5 secondes ou moins, par exemple, 2 secondes ou moins, par exemple, 1 seconde ou moins.
